# EUROPEAN PATENT APPLICATION

(11) **EP 4 512 320 A1**
(43) Date of publication of application: **26.02.2025**
(21) Application number: 22965539.4
(22) Date of filing: 17.11.2022
(51) Int. Cl.: A61B 5/024

(54) **WEARABLE DEVICE AND HEART MONITORING SYSTEM**

(71) Applicant: Shenzhen Shokz Co., Ltd., Shenzhen, Guangdong 518108 (CN)
(72) Inventor: ZHOU, Xin, Shenzhen, Guangdong 518108 (CN); ZHANG, Yuxiang, Shenzhen, Guangdong 518108 (CN); LIU, Jia, Shenzhen, Guangdong 518108 (CN); NING, Yixuan, Shenzhen, Guangdong 518108 (CN); SU, Lei, Shenzhen, Guangdong 518108 (CN); LI, Meiqi, Shenzhen, Guangdong 518108 (CN); ZHU, Huijin, Shenzhen, Guangdong 518108 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2022/132671
(87) International publication number: WO 2024/103357

(57) **Abstract**

A wearable device comprising at least two electrodes and a wearable structure. The at least two electrodes are configured to fit against the skin of a human body to collect an electrocardiogram (ECG) signal of the human body, and the wearable structure is configured to carry the at least two electrodes and fit the at least two electrodes to a waist region of the human body. The at least two electrodes are spaced apart on the wearable structure, and disposed on two sides of a mid-sagittal plane of the human body when the human body wears the wearable structure.

## Description

### TECHNICAL FIELD

The present disclosure relates to the field of wearable devices, and in particular, to wearable devices and cardiac monitoring systems.

### BACKGROUND

With the focus on scientific exercise and physical health, there is an increasing demand for real-time monitoring of human physiological signals. The ECG signal, as an important physiological signal of the human body, reflects the state of the human heart, and certain heart diseases (e.g., angina pectoris, coronary heart disease, etc.), may be examined or prevented by monitoring the ECG signal. Monitoring of the ECG signal mainly relies on electrodes, and by integrating electrodes into a garment and fitting the electrodes to the skin of a user, real-time monitoring and analysis of ECG signals of the user may be performed. Currently, most ECG monitoring is realized by integrating the electrodes on a top garment to fit a chest region of the user, which often causes discomfort to the user (especially those who are exercising), e.g., the constriction on the chest may affect the user's breathing, and the quality of the collected ECG signal is difficult to be guaranteed, e.g., the ECG signal may be interfered with by a relatively severe motion artifact, which affects the monitoring and analyzing results.

It is therefore desirable to provide a wearable device for high-quality real-time monitoring of the ECG signal of the user while ensuring that the user has good wearing comfort.

### SUMMARY

One of the embodiments of the present disclosure provides a wearable device comprising at least two electrodes and a wearable structure. The at least two electrodes are configured to fit against the skin of a human body to collect an electrocardiogram (ECG) signal of the human body, and the wearable structure is configured to carry the at least two electrodes and fit the at least two electrodes to a waist region of the human body. The at least two electrodes are spaced apart on the wearable structure, and disposed on two sides of a mid-sagittal plane of the human body when the human body wears the wearable structure.

In some embodiments, the at least two electrodes include a first electrode and a second electrode, and when the human body wears the wearable structure, the first electrode and the second electrode are fitted to iliac positions on the two sides of the mid-sagittal plane of the human body, respectively.

In some embodiments, when the human body wears the wearable structure, the first electrode and the second electrode are fitted to anterior superior iliac spine positions on the two sides of the mid-sagittal plane of the human body, respectively.

In some embodiments, when the human body wears the wearable structure, the first electrode and the second electrode are fitted to posterior superior iliac spine positions on the two sides of the mid-sagittal plane of the human body, respectively.

In some embodiments, the at least two electrodes include a first electrode and a second electrode, and when the human body wears the wearable structure, the first electrode and the second electrode are fitted to lower back positions on the two sides of the mid-sagittal plane of the human body, respectively.

In some embodiments, the at least two electrodes include a first electrode and a second electrode, and when the human body wears the wearable structure, the first electrode and the second electrode are fitted to abdomen regions on the two sides of the mid-sagittal plane of the human body, respectively.

In some embodiments, when the human body wears the wearable structure, the first electrode and the second electrode are disposed symmetrically with respect to the mid-sagittal plane of the human body.

In some embodiments, the wearable structure has a first extension direction and a second extension direction, the second extension direction being perpendicular to the first extension direction, the first electrode and the second electrode are spaced apart along the first extension direction of the wearable structure, a dimension of the first electrode or the second electrode in the first extension direction is in a range of 5 mm ~ 50 mm, and a dimension of the first electrode or the second electrode in the second extension direction is in a range of 5 mm ~ 50 mm.

In some embodiments, the at least two electrodes protrude from a surface of the wearable structure surrounding the at least two electrodes.

In some embodiments, the at least two electrodes include a first electrode, a second electrode, and a reference electrode, the first electrode and the second electrode are spaced apart and disposed on a surface of the wearable structure that is close to the skin of the human body, and the reference electrode is disposed between the first electrode and the second electrode.

In some embodiments, a dimension of the reference electrode along a second extension direction of the wearable structure is not less than a dimension of the first electrode or the second electrode along the second extension direction of the wearable structure.

In some embodiments, the dimension of the reference electrode along the second extension direction is in a range of 5 mm ~ 80 mm.

In some embodiments, the wearable device further comprises a circuit structure configured to process the ECG signal collected by the at least two electrodes. The circuit structure is disposed on a side of the wearable structure away from the at least two electrodes, and the at least two electrodes are electrically connected to the circuit structure via a wire.

In some embodiments, the circuit structure includes a contact impedance measurement circuit configured to obtain contact impedance when the at least two electrodes are fitted to a surface of the skin.

In some embodiments, the wearable device further comprises a fixing seat. The fixing seat includes a female seat and a sub-seat, the female seat is connected to the wearable structure, the sub-seat is detachably connected to the female seat, and the circuit structure is located within the sub-seat.

In some embodiments, the wearable device further comprises a sensor module configured to collect movement data of the human body, and the sensor module is communicatively connected to the circuit structure.

In some embodiments, the wearable structure is an elastic strap-like structure, a first Velcro patch is provided on a side of the wearable structure in contact with the human body, a second Velcro patch is provided on a side of each of the at least two electrodes away from a side where the electrode is fitted to the human body, and the each of the at least two electrodes is detachably connected with the wearable structure via the first Velcro patch and the second Velcro patch.

One of the embodiments of the present disclosure provides a cardiac monitoring system comprising the wearable device as described in any of the above embodiments, a heart rate feature extraction module, an analysis module, and a terminal device. The heart rate feature extraction module is configured to extract a heart rate feature of the human body based on the ECG signal. The analysis module is configured to match the heart rate feature with a database of heart rate features to obtain a heart rate analysis result. The terminal device is configured to receive and display the heart rate analysis result.

One of the embodiments of the present disclosure provides a cardiac monitoring system comprising the wearable device described in any of the foregoing embodiments, a trained machine learning model, and a terminal device. The wearable device is configured to acquire an electrocardiogram (ECG) signal of a human body. The trained machine learning model is configured to output a heart rate analysis result based on the ECG signal as an input. The terminal device is configured to receive and display the heart rate analysis result.

In some embodiments, the terminal device includes a processor and a prompting module. The processor is configured to issue a command based on the heart rate analysis result, and control the prompting module to send out a prompting message, make a phone call, or send a message.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering denotes the same structure, wherein:
FIG. 1 is a schematic diagram of a wearable device according to some embodiments of the present disclosure;
FIG. 2 is a schematic diagram of a structure of a wearable device according to some embodiments of the present disclosure;
FIG. 3 is a schematic diagram of a structure of a wearable device according to some embodiments of the present disclosure;
FIG. 4 is a schematic diagram of a human body;
FIG. 5 is a schematic diagram of an ilium in a waist region of a human body;
FIG. 6 is a schematic diagram of a structure of a wearable device according to some embodiments of the present disclosure;
FIG. 7 is a schematic diagram of waveforms of ECG signals collected, in a time domain during a test, by a first electrode and a second electrode at different locations on a human body, according to some embodiments of the present disclosure;
FIG. 8 is a schematic diagram of a processed waveform of an ECG signal collected in a time domain at anterior superior iliac spine positions on two sides of a mid-sagittal plane of a human body by a first electrode and a second electrode according to some embodiments of the present disclosure;
FIG. 9 is a schematic diagram of a conventional ECG;
FIG. 10 is a schematic diagram of a structure of a wearable device according to some embodiments of the present disclosure;
FIG. 11 is a schematic diagram of a circuit structure of a wearable device according to some embodiments of the present disclosure;
FIG. 12 is a schematic diagram of a frequency response curve of a circuit structure according to some embodiments of the present disclosure;
FIG. 13 is a comparison diagram of an actual frequency response curve versus a simulated frequency response curve of a circuit structure according to some embodiments of the present disclosure;
FIG. 14 is a schematic diagram of a structure of a fixing seat according to some embodiments of the present disclosure;
FIG. 15 is a schematic diagram of a cross-sectional view of a sub-seat according to some embodiments of the present disclosure;
FIG. 16 is a schematic diagram of a waveform of ECG signals of an experimental subject collected over a continuous period of time at different pacing speeds by a wearable device according to some embodiments of the present disclosure;
FIG. 17 is an enlarged view of the waveform of the ECG signals shown in FIG. 16 from about 1.7 minutes to 2.7 minutes;
FIG. 18 is an enlarged view of the waveform of the ECG signals shown in FIG. 16 from about 5.8 minutes to 7 minutes;
FIG. 19 is an enlarged view of the waveform of the ECG signals shown in FIG. 16 from about 9.8 minutes to 10.4 minutes;
FIG. 20 is a block diagram of a structure of a cardiac monitoring system according to some embodiments of the present disclosure; and
FIG. 21 is a block diagram of a structure of a cardiac monitoring system according to some embodiments of the present disclosure.

### DETAILED DESCRIPTION

In order to provide a clearer understanding of the technical solutions of the embodiments described in the present disclosure, a brief introduction to the drawings required in the description of the embodiments is given below. It is evident that the drawings described below are merely some examples or embodiments of the present disclosure, and for those skilled in the art, the present disclosure may be applied to other similar situations without exercising creative labor. Unless otherwise indicated or stated in the context, the same reference numerals in the drawings represent the same structures or operations.

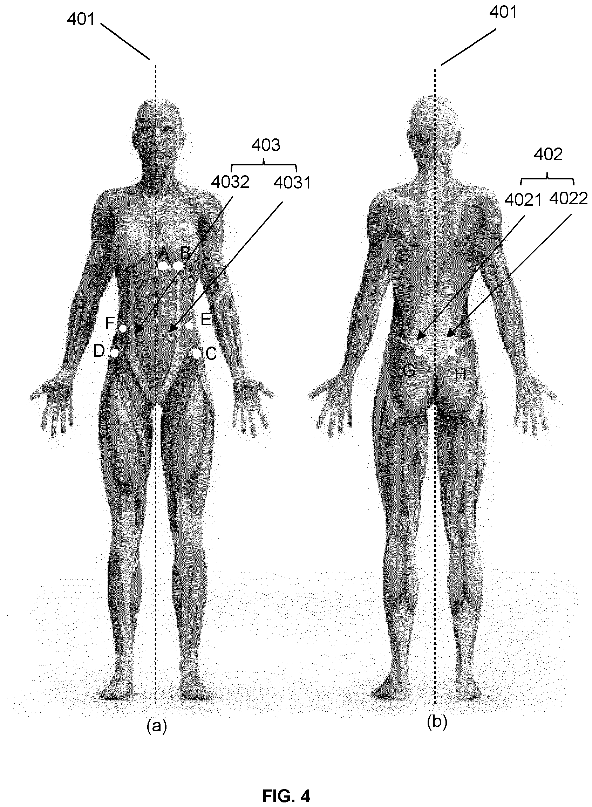

Embodiments of the present disclosure provide a wearable device, which may include at least two electrodes and a wearable structure. The at least two electrodes may be configured to fit against a skin of a human body to collect an electrocardiogram (ECG) signal of the human body, and the wearable structure may be configured to carry the at least two electrodes and fit the at least two electrodes to different positions of a waist region of the human body. In some embodiments, the at least two electrodes are spaced apart on the wearing structure, and the at least two electrodes are disposed on two sides of a mid-sagittal plane of the human body when the human body wears the wearing structure, which on one hand increases a distance difference from the heart to the at least two electrodes to increase a strength of the ECG signal associated with the distance difference, and on the other hand is conducive to reducing the interference of a motion artifact to the ECG signal, and thus can improve the quality of the collected ECG signal. There is little muscle in the waist region of the human body, especially at the ilium (e.g., the anterior side of the ilium), and there is almost no muscle covering this skeletal region. In some embodiments, when the human body wears the wearable structure, the at least two electrodes may be located at the ilium on the two sides of the mid-sagittal plane of the human body respectively, in order to minimize the interference of an electromyographic (EMG) signal with the ECG signal, and to ensure that the collected ECG signal is of a good quality. In addition, the perception sensitivity of the waist region to external objects or stimuli is lower compared to the perception sensitivity of other locations such as the chest, and arranging the electrodes in the waist region of the user allows the user to have a better sense of comfort when wearing the wearable device provided in the embodiments of the present disclosure, and even when the user is in a state of motion, a relatively high quality ECG signal may be collected without affecting the user's sense of experience. It may be understood that the ECG signal of the human body refers to bioelectrical changes induced by the heart beating being reflected to a surface of the human body through electrically conductive tissues and bodily fluids around the heart, so as to cause different parts of the surface of the human body to form different potential difference signals. The waist region of the human body refers to a portion of the human body between the ribs above the crotch, which is distributed on two sides of the spine, and specifically may include the abdominal and iliac portions of the human body. The mid-sagittal plane of a human body refers to a plane that passes through a median line of the body (e.g., the median line 401 shown in FIG. 4) and divides the body into two equal or approximately equal parts. It should be noted that the need for early warning of cardiac risks by a patient or an ordinary person may be fulfilled by wearing the wearable device provided in embodiments of the present disclosure. For example, the wearable device may provide a cardiac risk warning to the user by judging a cardiac condition of the patient or the user based on the collected ECG signal. In addition, the need for exercise and fitness guidance may be met by wearing the wearable device provided in embodiments of the present disclosure. For example, the wearable device may collect ECG signals and exercise data of the user, formulate a scientific exercise plan for the user based on the ECG signals of the user when exercising and the corresponding exercise data, and guide the user to exercise.

The wearable device provided in the embodiments of the present disclosure will be described in detail below in connection with the accompanying drawings.

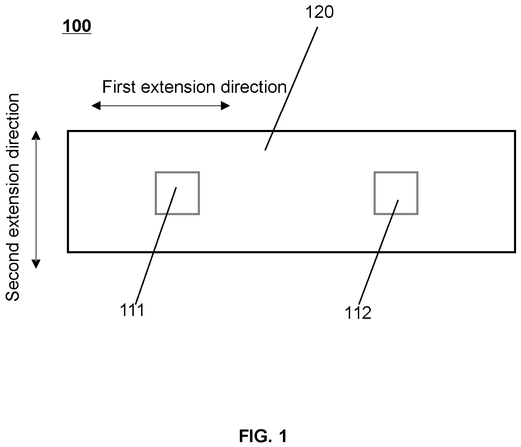

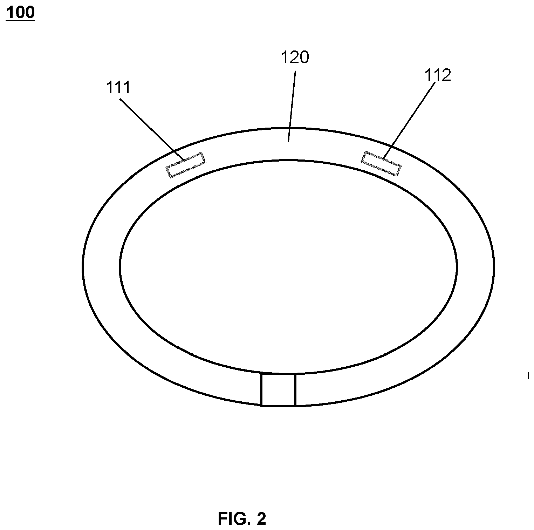

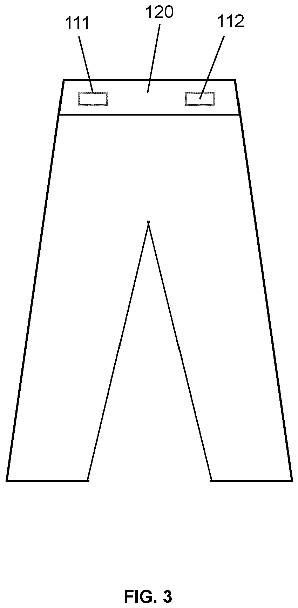

FIG. 1 is a schematic diagram of a wearable device according to some embodiments of the present disclosure. FIGs. 2 and 3 are schematic diagrams of structures of a wearable device according to some embodiments of the present disclosure.

Referring to FIG.1 ~ FIG.3, a wearable device 100 includes a first electrode 111, a second electrode 112, and a wearable structure 120.

The first electrode 111 and the second electrode 112 are configured to fit against the skin of a human body to collect an electrocardiogram (ECG) signal of the human body. In some embodiments, the first electrode 111 and the second electrode 112 may be fitted to different parts of the human body, wherein the part to which the first electrode 111 is fitted has a first potential and the part to which the second electrode 112 is fitted has a second potential. A difference between the first potential and the second potential may reflect an ECG signal of the human body.

The wearable structure 120 is configured to carry the first electrode 111 and the second electrode 112 and fit the first electrode 111 and the second electrode 112 to a waist region of the human body. The first electrode 111 and the second electrode 112 may be spaced apart on the wearable structure 120 when the human body wears the wearable structure 120. The first electrode 111 and the second electrode 112 may be disposed on two sides of a mid-sagittal plane of the human body to detect potentials (e.g., the first potential and the second potential) on the two sides of the mid-sagittal plane of the human body respectively, and determine the ECG signal based on the difference between the potentials on the two sides of the mid-sagittal plane of the human body. In some embodiments, as shown in FIG. 1, the wearable structure 120 has a first extension direction and a second extension direction perpendicular to the first extension direction, and the first electrode 111 and the second electrode 112 may be spaced apart along the first extension direction of the wearable structure. In some embodiments, the first extension direction of the wearable structure 120 may be a circumferential direction in which the wearable structure 120 is worn by the human body in the waist region of the human body.

In some embodiments, the wearable structure 120 may be configured as an elastic strap-like structure, and the first electrode 111 and the second electrode 112 may be spaced apart on the strap-like structure along an extension direction (or referred to as a length direction) of the strap-like structure. The user may tie the strap-like structure to his or her waist region or secure the strap-like structure to his or her waist region through a snap fastener member, so that the first electrode 111 and the second electrode 112 are respectively located on the two sides of the mid-sagittal plane of the human body. The first extension direction of the wearable structure 120 is a direction of extension of the strap-like structure. In some embodiments, the wearable structure 120 may be configured as a belt illustrated in FIG. 2, and the first electrode 111 and the second electrode 112 may be spaced apart and disposed on a surface of the belt that is fit to the skin of the human body. The first extension direction of the wearable structure 120 is a length direction of the belt after it is unfolded. In some embodiments, two ends of the belt may be combined using, for example, Velcro or a buckle, to facilitate the user's wearing or taking off. Configuring the wearable structure 120 as the elastic strap-like structure ensures better wearing comfort for the user and makes the wearable structure 120 resistant to washing. In some embodiments, the wearable structure 120 may also be configured as pants (e.g., shorts, pants, rompers, etc.) illustrated in FIG. 3. The first electrode 111 and the second electrode 112 may be disposed at intervals along a circumferential direction of a waistband of the pants on an inside of the waistband of the pants, so that when the human body wears the pants, the first electrode 111 and the second electrode 112 at the waistband of the pants may be disposed on the two sides of the mid-sagittal plane of the human body, respectively. In some embodiments, the wearable structure 120 may also be configured as a skirt, and the first electrode 111 and the second electrode 112 may be spaced apart along a circumferential direction of a waistline of the skirt and disposed on an inner side of the waistline, such that the first electrode 111 and the second electrode 112 are disposed on the two sides of the mid-sagittal plane of the human body, respectively. The first extension direction of the wearing structure 120 is a length direction of the waistband of the pants or the skirt after the waistline is unfolded along a circumferential direction of the waistline.

In some embodiments, the first electrode 111 and the second electrode 112 in the wearable device 100 may also be detachably coupled to the wearable structure 120. For example, the wearable structure 120 is the strap-like structure or pants, and a side of the first electrode 111 and the second electrode 112 that is not in contact with the skin may be connected to the wearable structure 120 through manners such as adhesion, snapping, or embedding. When the user measures the ECG signal, the first electrode 111 and the second electrode 112 are mounted on the strap-like structure or the waistband of pants, and positions of the first electrode 111 and the second electrode 112 on the wearable structure 120 may be adjusted according to a body shape (e.g., height, weight, waist circumference, etc.) of the user to accommodate users of different body shapes. When the user needs to clean the wearable structure 120 (e.g., the strap-like structure or the pants), components such as the first electrode 111 and the second electrode 112 may be removed from the wearable structure 120 to prevent damage to the components such as the first electrode 111 and the second electrode 112 during the cleaning process. In some embodiments, a side of the wearable structure 120 (e.g., the strap-like structure or the waistband of the pants) that is fitted to the user may be provided with a first Velcro patch, which may be distributed along the extension direction of the strap-like structure or the circumferential direction of the waistband of the pants, and a second Velcro patch is provided on a side of each of the first electrode 111 and the second electrode 112 away from a side where the electrode is fitted to the human body. The second Velcro patch may be bonded to the first Velcro patch so that the first electrode 111 and the second electrode 112 are detachably connected to the wearable structure 120. In some embodiments of the present disclosure, the first Velcro patch may cover a side of the wearable structure 120 that abuts the user. When the first Velcro patch is an integral structure, it affects the elasticity of the wearable structure 120, resulting in a poor wearable experience for the user. In some embodiments, a plurality of first Velcro patches may be provided, and the plurality of first Velcro patches may be sequentially stitched on the side of the wearable structure 120 that contacts the user. At this time, gaps between the plurality of first Velcro patches allow the wearable structure 120 to maintain its elasticity while also facilitating adjustments to the positions of the first electrode 111 and the second electrode 112 on the wearable structure 120, making it suitable for users of different body shapes. In some embodiments, the plurality of first Velcro patches may also be distributed at intervals along the extension direction of the strip-like structure or around the waistband of the pants. For example, a length of each first Velcro patch may be 4 cm, and a spacing between two adjacent patches may be 1 cm. In some embodiments, the first Velcro patch or the second Velcro patch may be made from a non-woven fabric material, which is relatively soft, so that when the user wears the wearable device 100, the first Velcro patch does not cause discomfort when in contact with the skin on the waist of the user, thereby enhancing the user's experience while wearing the device. In some embodiments, the wearable device 100 may include a plurality of sets of electrode modules (e.g., the first electrode 111 and the second electrode 112). Each set of electrode modules is provided with a wire. One end of the wire connects to the first electrode 111 or the second electrode 112 within the electrode module, while the other end has a connector that may be electrically connected to a circuit structure of the wearable device 100, such as a port on a circuit board of the circuit structure designed to match the connector. The user may replace different electrode modules based on their individual needs. In some embodiments, sizes of the plurality of electrode modules may be the same or different. For illustrative purposes only, the first electrode or the second electrode in the electrode module may have dimensions of 1 cm * 1 cm, 1 cm * 2 cm, 1 cm * 3 cm, 1 cm * 4 cm, 1 cm * 5 cm, 2 cm * 2 cm, 2 cm * 3 cm, 2 cm * 4 cm, 2 cm * 5 cm, 3 cm * 3 cm, 3 cm * 4 cm, 3 cm * 5 cm, 4 cm * 4 cm, 4 cm * 5 cm, or 5 cm * 5 cm. It should be noted that the first electrode or the second electrode in the electrode module is not limited to the dimensions enumerated above, but may also be of other dimensions.

In some embodiments, the first electrode 111 or the second electrode 112 may be fitted to the skin of the human body in the form of a flexible patch, which may be a regular shape such as a circle, an ellipse, a rectangle, a rhombus, or other irregular shapes. In practice, a shape of the first electrode 111 or the second electrode 112 may be designed according to a shape of a bone under the skin of the waist region to which the first electrode 111 or the second electrode 112 is fitted. In some embodiments, the first electrode 111 or the second electrode 112 may be an electrode made of a single material, such as a metal fabric electrode, a conductive silicon electrode, a hydrogel electrode, a metal electrode, or the like. Preferably, the first electrode 111 or the second electrode 112 may be a metallic fabric electrode and a conductive silicon electrode. More preferably, the first electrode 111 or the second electrode 112 may be a metal fabric electrode, which has lower resistivity, impedance, and contact impedance with the skin. A smaller the contact impedance between the first electrode 111 or the second electrode 112 and the skin is favorable to reducing the interference of a motion artifact with the ECG signal collected by the first electrode 111 and the second electrode 112. In some embodiments, the first electrode 111 or the second electrode 112 may protrude from a surface of the wearable structure 120 surrounding the first electrode 111 or the second electrode 112, which can provide prepressure to the electrode, facilitating better conformity of the first electrode 111 or the second electrode 112 to the skin for accurate ECG signal collection. In some embodiments, a height of the first electrode 111 or the second electrode 112 protruding with respect to the surface of the wearable structure 120 surrounding the electrode depends on a thickness of the first electrode 111 or the second electrode 112. The thickness of the first electrode 111 or the second electrode 112 may be a dimension of the first electrode 111 or the second electrode 112 in a direction perpendicular to the surface of the wearable structure 120 surrounding the electrode. The thickness of the first electrode 111 or the second electrode 112 is related to the material of the electrode. For example, in some embodiments, when using a metal fabric electrode to collect ECG signals, the thickness of the metal fabric electrode may be in a range of 10 µm to 5 mm. Preferably, the thickness of the metal fabric electrode may be in a range of 100 µm to 3 mm. More preferably, the thickness of the metal fabric electrode may be in a range of 500 µm to 2 mm. In some embodiments, the first electrode 111 or the second electrode 112 may also be an electrode formed by layering different materials, such as an electrode composed of metallic fabric and conductive silicone. This design not only ensures a small contact impedance with the skin but also provides advantages such as skin-friendliness, wash resistance, and abrasion resistance due to the conductive silicone in contact with the skin, thereby preventing discomfort caused by the electrode's contact with the human body.

In some embodiments, a dimension of the first electrode 111 or the second electrode 112 in the first extension direction or the second extension direction may be as large as possible, provided the dimension does not exceed a dimension of the wearable structure 120 in the first or second extension direction. This ensures that the first electrode 111 or the second electrode 112 has a relatively large contact area with the skin, thereby reducing the contact impedance between the electrode and the skin. Consequently, this helps minimize motion artifacts affecting ECG signals and enhances the intensity of the ECG signals collected by the first electrode 111 and the second electrode 112. Additionally, the relatively large dimension of the first electrode 111 or the second electrode 112 in the first or second extension direction makes the first electrode 111 or the second electrode 112 less susceptible to deformation and displacement during wear, which is beneficial for reducing motion artifacts and improving the quality of the ECG signals. Furthermore, the relatively large dimension of the first electrode 111 or the second electrode 112 in the first or second extension direction ensures that the first electrode 111 or the second electrode 112 does not completely detach from the skin during various movements or folds, thus ensuring consistent ECG signal collection. In some embodiments, the dimension of the first electrode 111 or the second electrode 112 in the first or second extension direction refers to a maximum dimension in the first or second extension direction. In some embodiments, the dimension of the first electrode 111 or the second electrode 112 in the first extension direction may be in a range of 5 mm to 50 mm, and the dimension of the first electrode 111 or the second electrode 112 in the second extension direction may be in a range of 5 mm to 50 mm. In some embodiments, the dimension of the first electrode 111 or the second electrode 112 in the first extension direction may be in a range of 10 mm to 45 mm, and the dimension of the first electrode 111 or the second electrode 112 in the second extension direction may be in a range of 10 mm to 45 mm. In some embodiments, the dimension of the first electrode 111 or the second electrode 112 in the first extension direction may be in a range of 15 mm to 40 mm, and the dimension of the first electrode 111 or the second electrode 112 in the second extension direction may be in a range of 15 mm to 40 mm. In some embodiments, the dimension of the first electrode 111 or the second electrode 112 in the first extension direction may be in a range of 20 mm to 30 mm, and the dimension of the first electrode 111 or the second electrode 112 in the second extension direction may be in a range of 20 mm to 30 mm.

In some embodiments, an isolation layer may be provided between the first electrode 111 or the second electrode 112 and a surface of the wearable structure 120 proximate to the skin of the human body. Specifically, the isolation layer is disposed on the surface of the wearable structure 120 surrounding the first electrode 111 or the second electrode 112. The isolation layer can prevent the wearing structure 120 from inadvertently activating the first electrode 111 or the second electrode 112 when it becomes wet, which may result in weak or inaccurate ECG signals being collected. In some embodiments, the isolation layer may be made from an insulating and waterproof material. The material of the isolation layer may include rubber, polymer materials, silicone, or any combination thereof.

The wearable device 100 provided in the embodiments of the present disclosure may be worn in the waist region of a human body, such that the first electrode 111 and the second electrode 112 are located on the two sides of the mid-sagittal plane of the human body, respectively, ensuring contact with the skin in the waist region on the two sides of the mid-sagittal plane to collect an ECG signal of the human body. This not only enables monitoring of the heart condition of the user wearing the device 100 but also ensures a high level of comfort. In some embodiments, the positions of the first electrode 111 and the second electrode 112 in the waist region of the human body relative to the mid-sagittal plane are related to the quality and intensity of the collected ECG signal. Below, a detailed description of the contact positions of the first electrode 111 and the second electrode 112 with the skin will be provided in conjunction with specific illustrations of the human body.

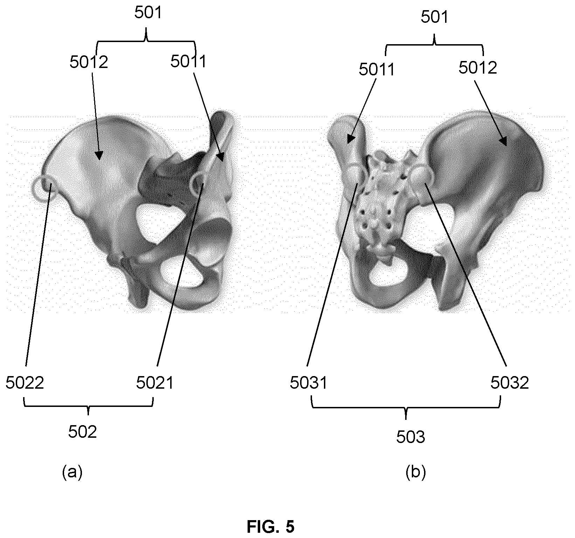

FIG. 4 is a schematic diagram of a human body, wherein (a) in FIG. 4 illustrates a front side of the human body and (b) in FIG. 4 illustrates a rear side of the human body. FIG. 5 is a schematic diagram of an ilium in a waist region of a human body, wherein (a) in FIG. 5 illustrates a front side of the ilium and (b) in FIG. 5 illustrates a rear side of the ilium.

As shown in (a) and (b) in FIG. 4, a mid-sagittal plane of the human body is a plane that passes through a median line 401 of the human body and divides the human body into two equal or approximately equal parts. The median line 401 of the human body may be determined based on a line connecting the tip of the nose to the midpoint between the nipples, a line from the midpoint between the nipples to the middle of the navel, or a line from the middle of the navel to the midpoint of the pubic symphysis. The waist region of the human body may be defined as a region between a lower end of the ribs and a lower end of the iliac crest, primarily including the abdomen and parts of the ilium. In some embodiments, when the human body wears the wearable structure 120, the first electrode 111 and the second electrode 112 may be fitted to the skin on the two sides of the mid-sagittal plane in the waist region. The mid-sagittal plane divides the waist region into a left waist region and a right waist region. In some embodiments, the first electrode 111 and the second electrode 112 may be fitted to the skin of the left waist region and the right waist region, respectively. In some embodiments, the first electrode 111 and the second electrode 112 can be symmetrically arranged with respect to the mid-sagittal plane, which ensures that motion artifacts at the locations where the first electrode 111 and the second electrode 112 are fitted have good consistency, thereby eliminating motion artifacts, such as by using a differential amplifier circuit to remove motion artifacts from ECG signals, and improving the signal quality. In some embodiments, by maintaining the consistency of the first electrode 111 and the second electrode 112, the motion artifacts between the positions to which the first electrode 111 and the second electrode 112 are fitted can be further improved in consistency, which is more conducive to the elimination of motion artifacts, resulting in a higher quality of the ECG signal. In some embodiments, the first electrode 111 and the second electrode 112 being consistent may include consistency in material, dimension (e.g., the dimension of the electrode in the first extension direction, the dimension of the electrode in the second extension direction, and the thickness of the electrode, etc.), pressure on the skin, etc., or any combination thereof. In some embodiments, the consistency of motion artifacts at the positions to which the first electrode 111 and the second electrode 112 are fitted to may be achieved by ensuring consistency in the elasticity of portions of the wearable structure 120 carrying the first electrode 111 and the second electrode 112, thereby facilitating the elimination of motion artifacts and improving ECG signal quality. It should be noted that, in some embodiments, the first electrode 111 and the second electrode 112 may also be asymmetrically disposed about the mid-sagittal plane of the human body. For example, when the user wears the wearable device, the first electrode 111 is located at the abdomen of the human body on one side of the mid-sagittal plane, and the second electrode 112 is located at the ilium of the human body on the other side of the midsagittal plane. As another example, when the user wears the wearable device, the first electrode 111 is located at the abdomen of the human body on one side of the midsagittal plane, and the second electrode 112 is located at the posterior waist region of the human body on the other side of the mid-sagittal plane. Additionally, the materials, dimensions, and pressure applied to the skin by the first electrode 111 and the second electrode 112 may be consistent or different.

In some embodiments, as shown in (a) and (b) in FIG. 5, when the human body is wearing the wearing structure 120, the first electrode 111 and the second electrode 112 are fitted to iliac 501 positions on the two sides of the mid-sagittal plane of the human body, respectively. As an exemplary illustration, the mid-sagittal plane of the human body may divide an ilium 501 into a left ilium 5011 and a right ilium 5012, and the first electrode 111 and the second electrode 112 may be fitted to skin regions corresponding to the left ilium 5011 and the right ilium 5012 respectively, to collect the ECG signal of the human body. In some embodiments, when the human body wears the wearing structure 120, fitting positions of the first electrode 111 and the second electrode 112 to the skin regions corresponding the left ilium 5011 and the right ilium 5012 may be symmetrical about the mid-sagittal plane of the human body, which can improve the consistency of motion artifacts at the fitting positions of the first electrode 111 and the second electrode 112, thereby facilitating the elimination of motion artifacts and improving the quality of the ECG signal. In some embodiments, the fitting positions of the first electrode 111 and the second electrode 112 to the skin regions corresponding to the left ilium 5011 and the right ilium 5012 may be asymmetrically disposed about the mid-sagittal plane of the human body.

In some embodiments, as shown in (a) in FIG. 5, when the human body is wearing the wearing structure 120, the first electrode 111 and the second electrode 112 may be fitted to anterior superior iliac spine 502 positions on the two sides of the midsagittal plane of the human body, respectively. The anterior superior iliac spine 502 may include a left anterior superior iliac spine 5021 and a right anterior superior iliac spine 5022 disposed on the left ilium 5011 and the right ilium 5012, respectively. As an exemplary illustration, when the human body wears the wearing structure 120, the first electrode 111 and the second electrode 112 may be fitted to skin regions corresponding to the left anterior superior iliac spine 5021 and the right anterior superior iliac spine 5022, respectively, to collect the ECG signal of the human body. Due to the relatively small amount of muscle at the positions of the left anterior superior iliac spine 5021 and the right anterior superior iliac spine 5022, even when the body is in motion, the interference of EMG signals with the electrocardiogram ECG signals collected by the first electrode 111 and the second electrode 112 is small, thereby ensuring a high-quality ECG signal. The left anterior superior iliac spine 5021 and the right anterior superior iliac spine 5022 are symmetrical relative to the mid-sagittal plane of the human body, resulting in consistent motion artifacts corresponding to the first electrode 111 and the second electrode 112, which aids in the elimination of motion artifacts and improves the quality of the ECG signal. Furthermore, there is a relatively large difference in distance between the left anterior superior iliac spine 5021 and the heart and distance between the right anterior superior iliac spine 5022 and the heart, leading to a relatively large potential difference between the left anterior superior iliac spine 5021 and the right anterior superior iliac spine 5022. The first electrode 111 and the second electrode 112 are respectively fitted to the skin regions corresponding to the left ilium 5011 and the right ilium 5012, which helps increase the strength of the collected ECG signal. Additionally, the left anterior superior iliac spine 5021 and the right anterior superior iliac spine 5022 are raised, which allows them to fit closely with the first electrode 111 and the second electrode 112, thereby ensuring that the electrodes are less likely to detach. The human body is less sensitive to the skin regions corresponding to the left anterior superior iliac spine 5021 and the right anterior superior iliac spine 5022, ensuring a good level of wearing comfort when the body wears the wearable structure 120.

In some embodiments, as illustrated in (b) in FIG. 5, the first electrode 111 and the second electrode 112 may be fitted to the posterior superior iliac spine 503 positions on the two sides of the mid-sagittal plane of the human body, respectively. The posterior superior iliac spine 503 includes a left posterior superior iliac spine 5031 located on the left ilium 5011 and a right posterior superior iliac spine 5032 located on the right ilium 5012, respectively. As an exemplary illustration, when the human body wears the wearing structure 120, the first electrode 111 and the second electrode 112 may be fitted to skin regions corresponding to the left posterior superior iliac spine 5031 and the right posterior superior iliac spine 5032, respectively, to collect the ECG signal of the human body. Due to the relatively small amount of muscle at the positions of the left posterior superior iliac spine 5031 and the right posterior superior iliac spine 5032, even when the human body is in a state of motion, the interference of EMG signals with the electrocardiogram ECG signals collected by the first electrode 111 and the second electrode 112 is small, thereby ensuring a high-quality ECG signal. Moreover, the left posterior superior iliac spine 5031 and the right posterior superior iliac spine 5032 are symmetrical about the mid-sagittal plane of the human body, and the motion artifacts have better consistency, which is conducive to the elimination of motion artifacts and improvement of the quality of the ECG signal.

In some embodiments, as shown in (b) in FIG. 4, when the human body wears the wearable structure 120, the first electrode 111 and the second electrode 112 may be fitted to lower back 402 positions on the two sides of the mid-sagittal plane of the human body, respectively. As an exemplary illustration, the mid-sagittal plane of the human body divides the lower back 402 into a left lower back 4021 and a right lower back 4022, and when the human body wears the wearable structure 120, the first electrode 111 and the second electrode 112 are respectively fitted to skin regions corresponding to the left lower back 4021 and the right lower back 4022 to collect the ECG signal. In some embodiments, when the human body wears the wearable structure 120, fitting positions where the first electrode 111 and the second electrode 112 are fitted to the skin regions corresponding to the left lower back 4021 and the right lower back 4022, respectively, may be symmetrical about the mid-sagittal plane of the human body, which can improve the consistency of the motion artifacts at the fitting positions of the first electrode 111 and the second electrode 112, thereby facilitating the elimination of the motion artifacts and improving the quality of the ECG signal. In some embodiments, the fitting positions where the first electrode 111 and the second electrode 112 are fitted to the skin regions corresponding to the left lower back 4021 and the right lower back 4022, respectively, may be asymmetric about the mid-sagittal plane of the body.

In some embodiments, as shown in (a) in FIG. 4, when the human body is wearing the wearable structure 120, the first electrode 111 and the second electrode may be fitted to abdomen 403 regions on the two sides of the mid-sagittal plane of the human body, respectively. As an exemplary illustration, the mid-sagittal plane of the human body divides the abdomen 403 into a left abdomen 4031 and a right abdomen 4032, and when the human body wears the wearable structure 120, the first electrode 111 and the second electrode 112 are respectively fitted to skin regions corresponding to the left abdomen 4031 and the right abdomen 4032 to collect the ECG signal. In some embodiments, when the human body wears the wearable structure 120, the fitting positions where the first electrode 111 and the second electrode 112 are fitted to the skin regions corresponding to the left abdomen 4031 and the right abdomen 4032, respectively, may be symmetrical about the mid-sagittal plane of the human body, which can improve the consistency of motion artifacts at the fitting positions of the first electrode 111 and the second electrode 112, thereby facilitating the elimination of motion artifacts and improving the quality of the ECG signal. In some embodiments, the fitting positions where the first electrode 111 and the second electrode 112 are fitted to the skin regions corresponding to the left abdomen 4031 and the right abdomen 4032, respectively, may be asymmetric about the mid-sagittal plane of the body.

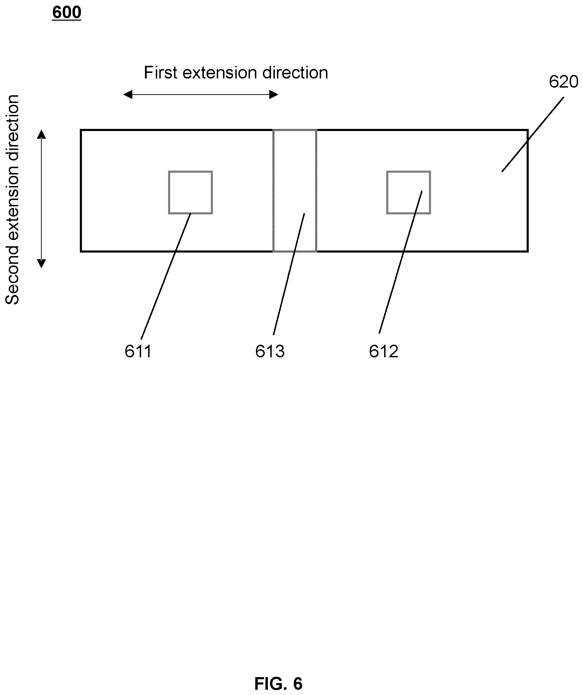

FIG. 6 is a schematic diagram of a structure of a wearable device according to some embodiments of the present disclosure.

In some embodiments, as shown in FIG. 6, a wearable device 600 includes a first electrode 611, a second electrode 612, a reference electrode 613, and a wearing structure 620. The first electrode 611 and the second electrode 612 may be spaced apart on a surface of the wearable structure 120 proximate to the skin of a human body, and the reference electrode 613 may be disposed between the first electrode 611 and the second electrode 612. The first electrode 611, the second electrode 612, and the wearing structure 620 are similar to the first electrode 111, the second electrode 112, and the wearing structure 120 in the wearable device 100. More descriptions regarding the first electrode 611, the second electrode 612, and the wearing structure 620 may refer to the relevant descriptions of the first electrode 111, the second electrode 112, and the wearing structure 120, and will not be repeated herein.

In some embodiments, when the human body wears the wearable structure 620, the first electrode 611 and the second electrode 612 may be fitted to two sides of a mid-sagittal plane of the human body, respectively, and the reference electrode 613 may fit against the skin of the human body. In some embodiments, the reference electrode 613 may provide a reference ground voltage for the first electrode 611 and the second electrode 612, thereby facilitating the subsequent use of circuitry (e.g., a differential amplifier) for processing a first potential corresponding to the first electrode 611 and a second potential corresponding to the second electrode 612. In some embodiments, when the human body wears the wearable device 600, the reference electrode 613 may be located on a lower back side of the body, the abdomen, or other locations. Preferably, when the human body wears the wearable device 600, the first electrode 611 and the second electrode 612 are arranged symmetrically with respect to the mid-sagittal plane of the human body. Accordingly, the reference electrode 613 may be located in a center (e.g., the lumbar spine and its vicinity) of the lower back side of the waist region of the human body, which enables the electrodes to be arranged symmetrically with respect to the mid-sagittal plane of the human body. This results in the value of a potential difference between the first potential corresponding to the first electrode 611 and the reference electrode 613 being approximately equal to the value of a potential difference between the second potential corresponding to the second electrode 612 and the reference electrode 613, facilitating subsequent processing of the circuitry (e.g., the differential amplifier). Additionally, the human body has fewer muscles on the lower back side, leading to a minor influence of EMG signals on the ECG signals. Furthermore, the lower back side of the human body has a lower sensitivity to perceptions of external objects or stimuli, ensuring a more comfortable wearing experience when using the wearable device 600. It should be noted that a count of reference electrodes 613 is not limited to the one shown in FIG. 6, but may be multiple. For example, the reference electrode 613 may include a first reference electrode and a second reference electrode. When the human body wears the wearable device 600, the first reference electrode and the second reference electrode may be located at a left lower back and a right lower back of the human body, respectively, and the first reference electrode and the second reference electrode may be arranged symmetrically with respect to the mid-sagittal plane of the body, so that the respective electrodes on the wearable device 600 are also symmetrically arranged about the mid-sagittal plane when the human body wears the wearable device 600. In some embodiments, the first reference electrode and the second reference electrode may also be arranged asymmetrically with respect to the mid-sagittal plane of the body. For example, both the first reference electrode and the second reference electrode may be located at the left lower back or the right lower back of the human body. As another example, the first reference electrode may be located at the left lower back away from the mid-sagittal plane of the human body, and the second reference electrode may be located at the right lower back close to the mid-sagittal plane of the human body. In some embodiments, the first reference electrode and the second reference electrode may be connected to each other by a wire to ensure that the two reference electrodes maintain the same potential, in which case the first reference electrode and the second reference electrode may be regarded as a single reference electrode, thereby increasing a degree of freedom of the wearable device 600 in terms of symmetry settings. Moreover, the first reference electrode and the second reference electrode are not limited to being located on the lower back side of the waist region of the human body, but may also be positioned in other regions such as the abdomen, the left and right waist sides, etc.

In some embodiments, a dimension of the reference electrode 613 along a second extension direction of the wearing structure 620 may be not smaller than a dimension of the first electrode 611 or the second electrode 612 along the second extension direction of the wearing structure 620 to enable the reference electrode 613 to fit against the human body. In some embodiments, the dimension of the reference electrode 613 in the second extension direction may range from 5mm to 80mm. In some embodiments, the dimension of the reference electrode 613 in the second extension direction may range from 10mm to 80mm. In some embodiments, the dimension of the reference electrode 613 in the second extension direction may range from 20mm to 80mm. In some embodiments, the dimension of the reference electrode 613 in the second extension direction may range from 30mm to 80mm. It should be noted that the dimension of the reference electrode 613 along the second extension direction of the wearing structure 620 may also be smaller than the dimension of the first electrode 611 or the second electrode 612 along the second extension direction of the wearing structure 620, as long as it is sufficient to provide a reference ground voltage.

In some embodiments, comparative tests of ECG signal collection may be conducted by performing ECG signal collection at different locations on the human body to study a relationship between the location of ECG signal collection and the ECG signal. An effect of the positions of the first electrode (e.g., the first electrode 111, the first electrode 611) and the second electrode (e.g., the second electrode 112, the second electrode 612) fitted to the skin of the human body on the ECG signals collected from the human body will be described below in connection with the accompanying drawings.

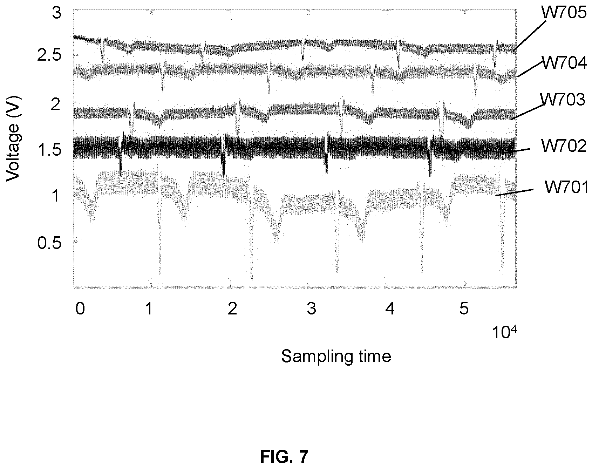

FIG. 7 is a schematic diagram of waveforms of ECG signals collected, in a time domain during a test, by a first electrode and a second electrode at different locations on a human body, according to some embodiments of the present disclosure. In FIG. 7, the horizontal coordinate represents the count of sampling points at a frequency of 8000 Hz, and the vertical coordinate represents the voltage (V) corresponding to the first electrode and the second electrode at different counts of sampling points, where the count of sampling points is a ratio of frequency to unit time (s). Waveform W701 represents an ECG signal collected in a time domain by the first electrode and the second electrode fitted to a chest region of the human body, wherein the first electrode and the second electrode are fitted to a position A and a position B respectively as shown in FIG. 4. Waveform W705 represents an ECG signal continuously collected at anterior superior iliac spine positions on two sides of the mid-sagittal plane of the human body to which the first electrode and the second electrode are respectively fitted. Waveform W702 represents an ECG signal collected at anterior superior iliac spine positions on the two sides of the mid-sagittal plane of the human body to which the first electrode and the second electrode are respectively fitted in the time domain, and the ECG signal is subjected to a high-pass filter processing at 1.6 Hz with a -3 dB point, wherein the first electrode and the second electrode are fitted to a position C and a position D shown in FIG. 4 respectively, and the position C and the position D correspond to the left anterior superior iliac spine 5021 and the right anterior superior iliac spine 5022 in FIG. 5 respectively. Waveform W703 represents an ECG signal continuously collected by the first electrode and the second electrode fitted to abdominal regions on the two sides of the mid-sagittal plane of the human body, wherein the first electrode and the second electrode are fitted to a position E and a position F shown in FIG. 4 respectively. Waveform W704 represents an ECG signal collected by the first electrode and the second electrode fitted positions of posterior superior iliac spines on the two sides of the mid-sagittal plane of the human body, wherein the first electrode and the second electrode are fitted to a position G and a position H shown in FIG. 4, and the position G and the position H correspond to the left posterior superior iliac spine 5031 and right posterior superior iliac spine 5032 respectively. It should be noted that each of the position A, the position B, the position C, the position D, the position E, the position F, the position G, and the position H shown in FIG. 4 represents a skin region having a certain area, and a shape and a size of the area of the skin region may depend on a shape and a size of the electrode fitted to the skin region.

By comparing waveforms W702, W703, W704, and W705 with waveform W701, it may be observed that the ECG signals collected in the time domain when the first electrode and the second electrode are respectively fitted to the positions of the anterior superior iliac spines, the posterior superior iliac spines, and the abdomen regions on the two sides of the midsagittal plane have a similar shape and a consistent cycle compared to the ECG signal collected from the chest region. For example, the sampling time from one peak to the next in the waveforms W701, W702, W703, W704, and W705 may be referred to as the cycle of the ECG signal. As shown in FIG. 7, the cycles of these waveforms are all approximately 1.2×10⁴, and the waveforms within a single cycle are essentially similar. This indicates that collecting ECG signals from the chest region is comparable to collecting them from the anterior superior iliac spines, the posterior superior iliac spines, and the abdomen regions on the two sides of the midsagittal plane. Moreover, considering the application in specific wearable devices, fitting electrodes to the chest region for ECG signal collection may cause discomfort to the user. Given the lower sensitivity of the waist region, the embodiments provided in the present disclosure suggest positioning the electrodes on the waist region (e.g., at the anterior superior iliac spines, the posterior superior iliac spines, or the abdomen regions, etc.) to collect ECG signals, thereby reducing or avoiding discomfort for the user.

In some embodiments, the interference of EMG signals of different positions on the human body on the ECG signals collected by the first and second electrodes may be studied through comparative tests. As an illustrative example, the EMG signals in the ECG signals collected from the positions of the anterior superior iliac spines, the posterior superior iliac spines, and the abdomen regions on the two sides of the midsagittal plane of the human body may be detected and compared. It may be concluded that when the first electrode and the second electrode are fitted to the positions of the anterior superior iliac spines or the posterior superior iliac spines on the two sides of the mid-sagittal plane of the human body, there are fewer EMG signals in the collected ECG signals compared to the scenario when the first electrode and the second electrode are fitted to the abdomen regions on the two sides of the mid-sagittal plane of the human body. In other words, collecting ECG signals from the anterior superior iliac spines or the posterior superior iliac spines on the two sides of the midsagittal plane results in less EMG interference, thus higher quality, because there are fewer muscles in these regions, leading to less EMG interference during ECG signal collection. Preferably, when the user wears the wearable device, the first electrode and second electrode may be respectively fitted to the anterior superior iliac spines or the posterior superior iliac spines on the two sides of the mid-sagittal plane of the human body.

In some embodiments, interference of motion artifacts at different locations of the human body on the ECG signals collected by the first electrode and the second electrode may be investigated by detecting the interference of the motion artifacts at different positions of the human body on the ECG signals through comparative tests. As an exemplary illustration, the interference of the first electrode and the second electrode may be investigated by detecting the interference of the motion artifacts of different positions of the human body on the ECG signals collected by the first electrode and the second electrode by fitting the first electrode and the second electrode to the positions of the anterior superior iliac spines, the posterior superior iliac spines, and the abdomen regions on the two sides of the mid-sagittal plane of the human body. It may be concluded that the motion artifacts in the ECG signals collected when the first electrode and the second electrode are respectively fitted to the positions of the anterior superior iliac spines on the two sides of the mid-sagittal plane of the human body are fewer compared to when the electrodes are attached to the positions of the posterior superior iliac spines and the abdomen regions on the two sides of the mid-sagittal plane. The ECG signals collected from the abdomen regions on the two sides of the mid-sagittal plane contain more motion artifacts. This is because, during movement, there is greater relative displacement between the electrodes and the fitting positions at the posterior superior iliac spines and the abdomen regions on the two sides of the midsagittal plane, which generates more motion artifacts. Conversely, the anterior superior iliac spines on the two sides of the mid-sagittal plane have a protrusion that ensures a snug fit with the electrodes, which reduces the relative displacement during movement and, thereby, diminishing the interference of motion artifacts on the ECG signals.

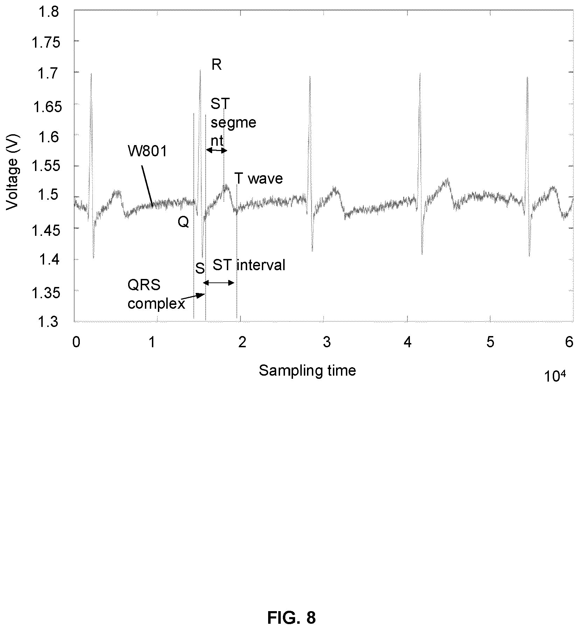

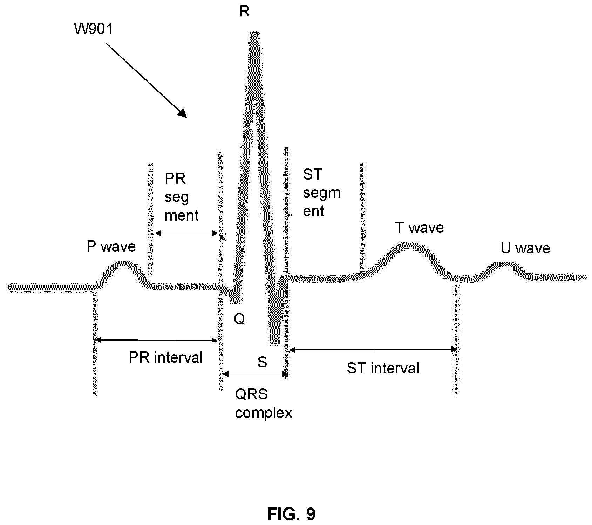

From FIG. 7, it may be seen that waveforms W701, W702, W703, W704, and W705 have a significant amount of power frequency and harmonic interference. This is due to a high level of interference introduced during the testing process. In practical applications, the suppression of power frequency and harmonics may be achieved through appropriate circuit routing, signal processing circuits, or algorithms to reduce or eliminate the harmonics and power frequency in the ECG signals collected by wearable devices. This allows relevant information to be extracted from the ECG signals, enabling the waveform to clearly display features such as a QRS complex and an ST segment, similar to traditional electrocardiograms, thereby facilitating the assessment of the condition of the heart based on these features. For more details, refer to Figures 8 and 9 and their associated descriptions.

FIG. 8 is a schematic diagram of a processed waveform of an ECG signal collected in a time domain at anterior superior iliac spine positions on two sides of a mid-sagittal plane of a human body by a first electrode and a second electrode according to some embodiments of the present disclosure. FIG. 9 is a schematic diagram of a conventional ECG. Waveform 901 illustrated in FIG. 9 reveals features such as a P wave, a QRS complex, an ST segment, a T wave, a PR interval, a U wave, or the like.

In some embodiments, the processing of the ECG signals collected in the time domain by the first and second electrodes positioned at the anterior superior iliac spine positions on the two sides of the midsagittal plane of the human body may include high-pass filtering at 1.6Hz with a -3dB point, notch filtering at frequencies of 50Hz, 150Hz, 250Hz, and 350Hz, and band-pass filtering with a frequency range of 0.5Hz to 400Hz. The waveform of the ECG signal collected in the time domain at the anterior superior iliac spine positions on the two sides of the mid-sagittal plane without any processing may be represented as the waveform W705 shown in FIG. 7. The ECG signal after high-pass filtering at 1.6Hz with a -3dB point may be represented as the waveform W702 in FIG. 7. The waveform of the ECG signal after all the aforementioned processing may be represented as the waveform W801 in FIG. 8. By comparing waveform W702, waveform W705, and waveform W801, it may be deduced that after the high-pass, notch, and band-pass filtering, the power frequency and harmonics are effectively suppressed. From the waveform W901 shown in Figure 9, it is evident that the waveform W801 clearly shows the QRS complex, ST segment, T wave, and ST interval. However, due to different ECG signal transmission paths, non-linear filtering circuits, the influence of filter cutoff points on the ECG signals, and partial common-mode ECG suppression by differential amplifiers, waveform W801 may not fully display features like the P wave, QRS complex, ST segment, T wave, PR interval, and U wave as waveform W901 does. Nevertheless, features such as the QRS complex, ST segment, T wave, and ST interval shown by waveform W801 are sufficient for recording heart rate and assessing cardiac condition. For example, a heart rate may be determined by measuring a time interval between two adjacent R waves. Additionally, if the level or down-sloping of the ST segment exceeds 0.05mV, it may indicate myocardial ischemia, possibly angina. If there is more than 0.1mV elevation in the ST segment with dynamic changes or a deep and wide Q wave, it suggests coronary artery disease and possibly acute myocardial infarction. Moreover, by analyzing the heights of the R and S waves along with changes in the ST segment and T wave, it is possible to detect whether the user has left ventricular hypertrophy. In some embodiments, the state of the human body may also be analyzed based on ECG signals. Merely by way of example, a heart rate variability (HRV) feature may be extracted from the ECG signals to analyze the state of the body. HRV refers to variations in heartbeats, which may be affected by factors such as physical activities and emotional states. Exemplarily, the emotional states may include stress levels, anxiety, focus, or the like. Techniques for analyzing the HRV may include, but are not limited to, time-domain, frequency-domain, and nonlinear analysis techniques. In some implementations, the HRV features may also be used to analyze cardiac diseases like coronary artery disease, myocardial infarction, heart failure, and arrhythmias, as these conditions may lead to reduced HRV.

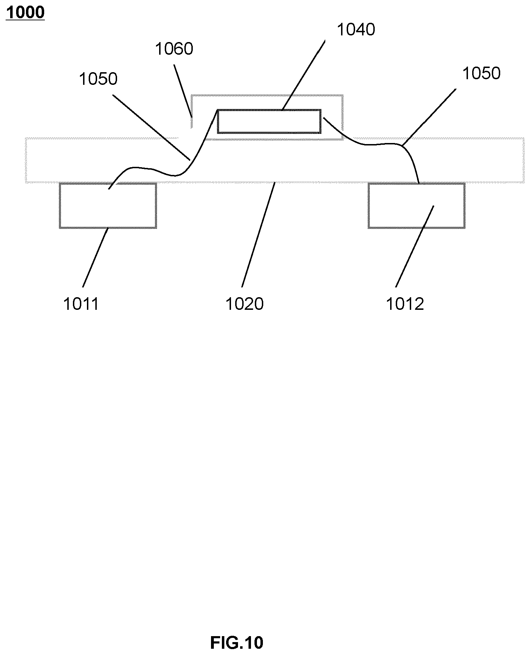

Based on the above, it may be seen that it is necessary to process the ECG signals collected by the first electrode and the second electrode in order to obtain ECG signals having practical application significance (e.g., recording heart rate, determining heart condition, etc.). Accordingly, the wearable device provided by the embodiments of the present disclosure needs to have a circuit structure that is configured to process the ECG signals collected by the first electrode and the second electrode, as described in detail in FIG. 10 and its related description.

FIG. 10 is a schematic diagram of a structure of a wearable device according to some embodiments of the present disclosure.

As shown in FIG. 10, in some embodiments, a wearable device 1000 includes a first electrode 1011, a second electrode 1012, a wearable structure 1020, and a circuit structure 1040. The circuit structure 1040 may be located on a side of the wearable structure 1020 facing away from the first electrode 1011 and the second electrode 1012, and the first electrode 1011 and the second electrode 1012 may be electrically connected via a wire 1050. The first electrode 1011, the second electrode 1012, and the wearable structure 1020 are similar to the first electrode 111, the second electrode 112, and the wearable structure 120 in the wearable device 100, respectively. More descriptions of the first electrode 1011, the second electrode 1012, and the wearable structure 1020 may be found in the relevant descriptions of the first electrode 111, the second electrode 112, and the wearable structure 120, and will not be repeated herein. In some embodiments, the wearable device 1000 may also include a reference electrode (not shown in the drawings) disposed between the first electrode 1011 and the second electrode 1022, and more description about the reference electrode may be referred to the relevant description of the wearable device 600, which will not be repeated here.

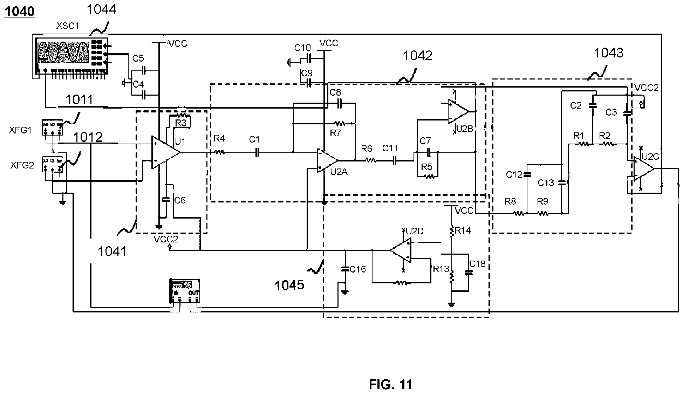

In some embodiments, when the first electrode 1011 and the second electrode 1012 collect an ECG signal, the ECG signal may be transmitted to the circuit structure 1040 via the wire 1050, and the circuit structure 1040 may process the received ECG signal, e.g., eliminating or suppressing a limit drift, a motion artifact, an EMG signal, a power frequency, a harmonic, etc., in the ECG signal, and amplify the ECG signal to obtain an ECG signal that is of high quality (e.g., a signal-to-noise ratio) and has practical applications. In some embodiments, the processing of the ECG signal by the circuit structure 1040 may include, but is not limited to, gain processing, differential amplification processing, low-pass filtering processing, spot high-pass filtering processing (e.g., high-pass filtering at 1.6Hz with a -3dB point ), notch filtering processing (e.g., notch processing filtering at frequencies of 50Hz, 150Hz, 250Hz, 350Hz), band-pass filtering processing (e.g., band-pass filtering with a filtering frequency of 0.5Hz~400Hz), or any combination thereof. The working principles of the circuit structure 1040 are illustrated below in connection with FIG. 11.

FIG. 11 is a schematic diagram of a circuit structure of a wearable device according to some embodiments of the present disclosure.

In some embodiments, the circuit structure 104 includes a secondary gain circuit 1041, an active band-pass filter 1042, a passive low-pass filter 1043, and a mode converter 1044, as illustrated in FIG. 11. The secondary gain circuit 1041 may be connected to the first electrode 1011 and the second electrode 1012 (e.g., via the wire 1050), and the secondary gain circuit 720, the active band-pass filter 730, the passive low-pass filter 740, and an analog-to-digital converter 750 are connected in turn.

The secondary gain circuitry 1041 may provide a common-mode rejection capability and provide a gain for an ECG signal input to circuit structure 1040 to increase a strength and a signal-to-noise ratio of the ECG signal, thereby enhancing the quality of the ECG signal. For example, an ECG signal rate is mainly distributed in a frequency band of 0.5 Hz to 400 Hz, and the secondary gain circuit 1051 may provide gain for the ECG signal in this frequency band, which improves the signal-to-noise ratio of the ECG signal. In some embodiments, the secondary gain circuit 1041 may provide a secondary gain of 1 to 1,000 times for the ECG signal. In some embodiments, the secondary gain circuit 1041 may be a separately configured amplification sub-circuit. In some embodiments, the circuit structure 1040 may also include other amplification sub-circuits (e.g., an amplification sub-circuit in the active band-pass filter 1042) capable of providing other gains (e.g., the first gain) to the ECG signal. In comparison to other amplification sub-circuits, the secondary gain circuit 1041 is located at a preceding stage, and the gain provided by the secondary gain circuit may be less than the gain provided by the other amplification sub-circuits. In some embodiments, the secondary gain circuitry may provide a secondary gain of 10 times. The magnitude of the secondary gain may be adjusted by adjusting a component parameter (e.g., a size of a capacitor resistor, etc.) in the secondary gain circuit.

In some embodiments, as shown in FIG. 11, the secondary gain circuit 1041 includes an operational amplifier U1, with a positive phase input and an inverted phase input of the operational amplifier U1 being connected to an electrode, respectively, to receive an ECG signal and/or other detection signals (e.g., signals for detecting contact impedance) as an input signal. A power supply terminal of the operational amplifier U1 is connected to power supply VCC, and a ground terminal of the operational amplifier U1 is grounded. A reference terminal of the operational amplifier U1 is connected to a virtual ground VCC2, and an output terminal of the operational amplifier U1 is configured to output a gained signal. The value of the gained signal may be equal to the value of a difference between the output terminal and the reference terminal of the operational amplifier U1. A first gain control terminal and a second gain control terminal of the operational amplifier U1 may be connected via a resistor R3. In some embodiments, there is a correspondence between the resistance value of the resistor R3 and the amplification of the operational amplifier U1. In some embodiments, the secondary gain provided by the secondary gain circuit 1041 to the input signal may be adjusted by adjusting a resistance of the resistor R3. Exemplarily, by increasing the resistance of the resistor R3, the secondary gain may be decreased. Conversely, by decreasing the resistance of the resistor R3, the secondary gain may be increased. In some embodiments, the operational amplifier U1 may be an amplifier, such as a differential amplifier or an instrumentation amplifier, such that the operational amplifier U1 may have a high input impedance property capable of reducing a change in the input signal in a circuit when the circuit is connected. In some embodiments, the secondary gain circuit 1041 has a high input impedance, which can reduce changes in signals that occur when the circuit is connected, making the collected signal more stable.

An input of the active band-pass filter 1042 may be coupled to the first electrode 1011 and the second electrode 1012, and the ECG signal and/or other detection signals collected by the first electrode 1011 and the second electrode 1012 may be designated as an input for filtering processing. In some embodiments, as shown in FIG. 11, the active band-pass filter 1042 may include operational amplifiers U2A-U2B, resistors R4, R6-R7, and capacitors C1, C7-C11. Wherein, a positive phase input of the operational amplifier U2A may be connected to a virtual ground VCC2. The inverted-phase input of the operational amplifier U2A may be connected to the series-connected capacitor C1 and the resistor R4, and the inverted-phase input of the operational amplifier U2A may be connected to the operational amplifier through the parallel-connected capacitor C8 and the resistor R7. The output of U2A is connected. The power supply terminal of the operational amplifier U2A is connected to the power supply VCC, and the ground terminal of the operational amplifier U2A is grounded. The inverting input of the operational amplifier U2B may be connected to the output of the operational amplifier U2A through the series-connected resistor R6 and the capacitor C11, and the positive input of the operational amplifier U2B may be connected to the virtual ground VCC2. The inverting input of the operational amplifier U2B may be connected to the output of the operational amplifier U2A through the capacitors C7 and R5 connected in parallel, and the power terminal and the ground terminal of the operational amplifier U2B are null. In some embodiments, the active band-pass filter 1042 is also configured to provide a first gain for the ECG signal and the detection signal. In some embodiments, the operational amplifier U2A and operational amplifier U2B of the active band-pass filter 1042 may provide a first gain for an input signal (e.g., an ECG signal and/or other detection signal). The magnitude of the first gain may be related to the parameters of resistors R4, R7, and capacitors C1, C8. In some embodiments, the first gain may be provided after the active band-pass filter 1042 has filtered the ECG signal and the detection signal. In some embodiments, the output of the operational amplifier U2B of the active band-pass filter 1042 may be configured to output the band-pass-filtered as well as partially-gained ECG signals and detection signals. Capacitor C10 and capacitor C9 are connected in parallel, with one end of both connected to the power supply VCC and the other end grounded. In some embodiments, the active band-pass filter 1042 may band-pass filter the ECG signals and the detection signals through resistors R4, R5-R7, and capacitors C1, C7-C8, C11. In some embodiments, the bandwidth frequency range of the active band-pass filter 1042 may be adjusted by adjusting component parameters of the aforementioned resistors R4, R5-R7, and capacitors C1, C7-C8, C11. It should be noted that the operational amplifiers U2A, U2B, U2D, and the follower U2C shown in FIG. 11 may be a single amplifier chip, i.e., the amplifier chip contains the operational amplifiers U2A, U2B, U2D, and follower U2C, wherein U2A, U2B, U2D, and follower U2C may share the power supply terminal and the ground terminal. In other embodiments, the operational amplifiers U2A, U2B, U2D, and follower U2C shown in FIG. 11 may also be operational amplifiers independent of each other, and each operational amplifier (follower) may have a power supply terminal and a ground terminal, respectively.

The passive low-pass filter 1043 may receive the ECG signal and/or other detection signals from the active band-pass filter 1042. After the ECG signal and/or other detection signals have been gained, high-frequency noise in the ECG signal and the detection signal is filtered out according to a second cutoff frequency to prevent the introduction of high-frequency noise during the gain process in order to improve signal quality. In some embodiments, the passive low-pass filter 1043 may include resistors R1-R2 and R8-R9, and capacitors C2-C3 and C12-C13, as shown in FIG. 11. The R8-R9, R2, and R1 are sequentially connected in series, and one end of each of the capacitors C2-C3 and C12-C13 is connected to a virtual ground VCC2. The other end of the capacitor C12 is connected to a connection point of the resistors R8-R9, the other end of the capacitor C13 is connected to a connection point of the resistors R9 and R2, the other end of the capacitor C2 is connected to a connection point of the resistors R2 and R1, and the other end of the capacitor C3 is connected to the resistor R1. In some embodiments, an end of the resistor R8 away from the resistor R9 may be configured to receive the gained ECG signal and detection signal, and an end of the resistor R1 away from the resistor R2 may be configured to output the low-pass filtered ECG signal and detection signal. In some embodiments, the passive low-pass filter 1043 includes four RC low-pass filtering units. Exemplarily, the passive low-pass filter 1043 may include capacitor C12-resistor R8, capacitor C13-resistor R9, capacitor C2-resistor R2, and capacitor C3-resistor R1. In some embodiments, the passive low-pass filter 1043 may utilize the foregoing RC low-pass filtering units for low-pass filtering of the detection signal and the ECG signal. In some embodiments, the second cutoff frequency of the passive low-pass filter 1043 may be adjusted by adjusting a component parameter in the foregoing RC low-pass filter units. In embodiments of the present disclosure, since the passive low-pass filter 1043 does not require an additional power supply (e.g., the power supply VCC), it allows the passive low-pass filter 1043 to introduce less aliasing noise, which can improve the quality of the ECG signal and the detection signal.

It should be noted that instead of providing gain (e.g., the second gain) for the input signals (e.g., the ECG signal and other detection signals after the first gain), the passive low-pass filter 1043 may utilize the amplification sub-circuit in the aforementioned active band-pass filter 1042 to provide the first gain for the ECG signal and the detection signal to achieve a gain function. In some embodiments, the gain function may also be realized by providing the second gain by way of a separate active amplification sub-circuit at the input of the passive low-pass filter 1043.

The analog-to-digital converter 1044 (e.g., the analog-to-digital converter XSC1 shown in FIG. 11) may receive the ECG signal and/or other detection signals from the active band-pass filter 1042 and perform an analog-to-digital conversion thereof. In some embodiments, the analog-to-digital converted ECG signal and/or other detection signals may also be processed by a filter to extract the ECG signal and/or other detection signals for subsequent evaluation of the ECG signal and/or other detection signals. In some embodiments, the analog-to-digital converter 1044 may sample the ECG signal and/or the other detection signals according to a sampling rate so as to generate a digital signal corresponding to the ECG signal and/or the other detection signals.

In some embodiments, as illustrated in FIG. 11, a follower U2C may be provided between the passive low-pass filter 1043 and the analog-to-digital converter 1044. The follower U2C may be configured to maintain a stable output voltage or current, and isolate the passive low-pass filter 1043 from the analog-to-digital converter 1044. A positive-phase input terminal of the follower U2C is coupled to an output of the passive low-pass filter 1043 for receiving the ECG signal and the detection signal, an inverted-phase input terminal of the follower U2C is connected to an output terminal of the follower U2C, and a power terminal and a ground terminal of the follower U2C are nulled.

In some embodiments, as shown in FIG. 11, the circuit structure may also include a power supply circuit 1045, the power supply circuit 1045 may be connected to a power supply VCC, and an output of the power supply circuit 1045 may be connected to a virtual ground VCC2 and provide power to the virtual ground VCC2. In some embodiments, when the voltage of the power supply VCC is 3.3V, the power supply circuit 1045 may provide a power supply of 1.65V or another power supply voltage within a dynamic range to the virtual ground VCC2.

In some embodiments, the power supply circuit 1045 may include an operational amplifier U2D, a positive-phase input terminal of the operational amplifier U2D is connected to the power supply VCC through a resistor R14, and the positive-phase input terminal of the operational amplifier U2D is also connected to ground through parallel-connected resistor R13 and capacitor C18. An inverted-phase input terminal of the operational amplifier U2D is connected to an output terminal of the operational amplifier U2D through a resistor R15, and the output terminal of the operational amplifier U2D is grounded through a capacitor C16. Exemplarily, an impedance value of the resistors R13- R14 may be 10 kΩ, an impedance value of the resistor R15 may be 300 Ω, a capacitance value of the capacitor C16 may be 10 µF, and a capacitance value of the capacitor C18 may be 10 µF.

In some embodiments, the circuit structure 1040 may include a contact impedance measurement circuit (not shown in the drawings), and the contact impedance measurement circuit may be configured to obtain contact impedance when the first electrode 1011 and the second electrode 1012 are fitted to a surface of the skin of a human body. As an exemplary illustration, when the human body wears the wearing structure 1020, the first electrode 1011 and the second electrode 1012 are fitted to the skin of the human body to collect, in addition to collecting an ECG signal of the human body, a detection signal that may reflect the magnitude of the contact impedance when the first electrode 1011 and the second electrode 1012 are fitted to the surface of the skin of the human body, and the detection signal may be inputted into the contact impedance measurement circuit to measure the contact impedance.

In some embodiments, factors such as a fitting condition between the electrodes and the skin, wetness of the skin surface, etc. may affect the magnitude of the contact impedance when the first electrode 1011 and the second electrode 1012 are fitted to the surface of the skin of the human body. For example, a relatively poor fit between the electrodes and the skin may result in a relatively large contact impedance when the first electrode 1011 and the second electrode 1012 are fitted to the surface of the skin of the human body; a relatively moist skin may result in a relatively small contact impedance when the first electrode 1011 and the second electrode 1012 are fitted to the surface of the skin of the human body. Thus, by using the contact impedance measurement circuit to measure the contact impedance, it is possible to monitor the fitting condition of the first electrode 1011 and the second electrode 1012 with the skin, sweat on the skin surface of the human body, a warm-up state of the human body, whether the wearable device is fitting properly, wear and tear of products (e.g., the first electrode 1011 and the second electrode 1012), or the like. In some embodiments, displacement due to relative motion between the first electrode 1011 and the second electrode 1012 and the skin to which they are fitted may cause the contact impedance of the first electrode 1011 and the second electrode 1012 to be altered when they are fitted to the surface of the skin of the human body. The change in the contact impedance may cause a motion artifact that interferes with the collection of the ECG signal by the first electrode 1011 and the second electrode 1012. Both the contact impedance and the motion artifact reflect motion information of the first electrode 1011 and the second electrode 1012 fitted to the skin, and it may be concluded that there is a correspondence between the contact impedance and the motion artifact. Therefore, when processing the ECG signal to eliminate the motion artifact in the ECG signal, the contact impedance measurement circuit may be configured to obtain the contact impedance when the first electrode 1011 and the second electrode 1012 are fitted to the surface of the skin of the human body, and then the motion artifact in the ECG signal may be sufficiently extracted according to the correspondence between the contact impedance and the motion artifact, so as to improve the quality of the ECG signal.

In some embodiments, the circuit structure 1040 may further include a differential amplification circuit, and the differential amplification circuit may be configured to eliminate the motion artifact in the ECG signal and improve the quality of the ECG signal. As an exemplary illustration, when the first electrode 1011 and the second electrode 1012 are symmetrically disposed with respect to the mid-sagittal plane of the human body and have a good congruence, positions where the first electrode 1011 and the second electrode 1012 that are fitted to the skin have consistent motion artifacts that may be eliminated by the differential amplification circuit. In some embodiments, the differential amplification circuitry may be integrated into the secondary gain circuit 1041, the active band-pass filter 1042, the passive circuit band-pass filter 1043, or the power supply circuit 1045, etc. The differential amplification circuit may be the operational amplifier U1, the operational amplifier U2A, or the operational amplifier U2B. In some embodiments, the differential amplification circuit may also be a circuit provided in the circuit structure 1040 independently of other circuits.

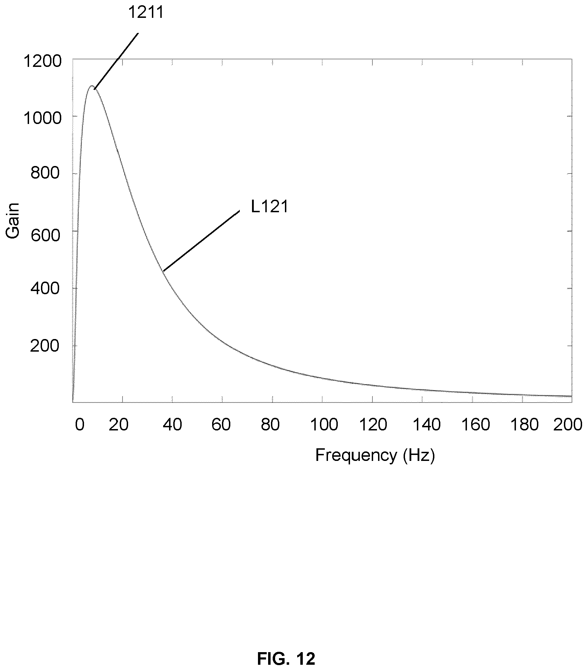

FIG. 12 is a schematic diagram of a frequency response curve of a circuit structure according to some embodiments of the present disclosure. Curve L121 represents a frequency response curve of the circuit structure 1040. Referring to the curve L121, it may be seen that the curve L121 has a frequency response peak 1211 at about 10 Hz, which indicates that the circuit structure 1040 has a high gain for an ECG signal at about 10 Hz, and is capable of suppressing an interference of a noise (e.g., a motion artifact, an EMG signal, a power frequency, harmonics, etc.) in the ECG signal. As a result, the ECG signal has a relatively high signal-to-noise ratio and a relatively high quality.

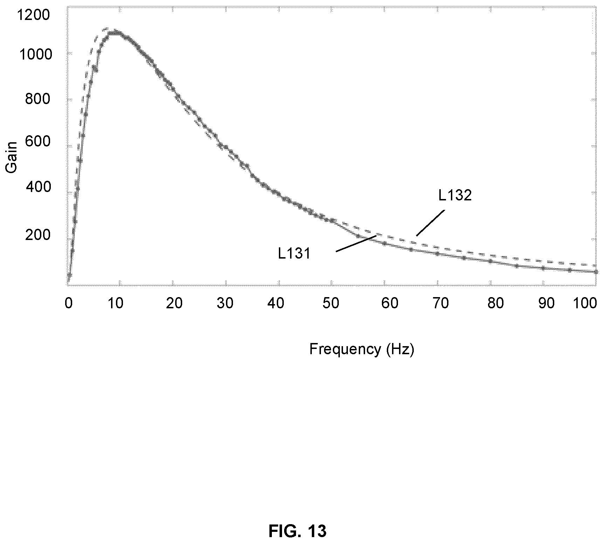

FIG. 13 is a comparison diagram of an actual frequency response curve versus a simulated frequency response curve of a circuit structure according to some embodiments of the present disclosure. Curve L131 represents an actual frequency response curve of the circuit structure 1040, and curve L132 represents a simulated frequency response curve of the circuit structure 1040. It may be seen that the consistency of curve L131 and curve L132 is high, which indicates that the circuit structure 1040 is stable and accurate.

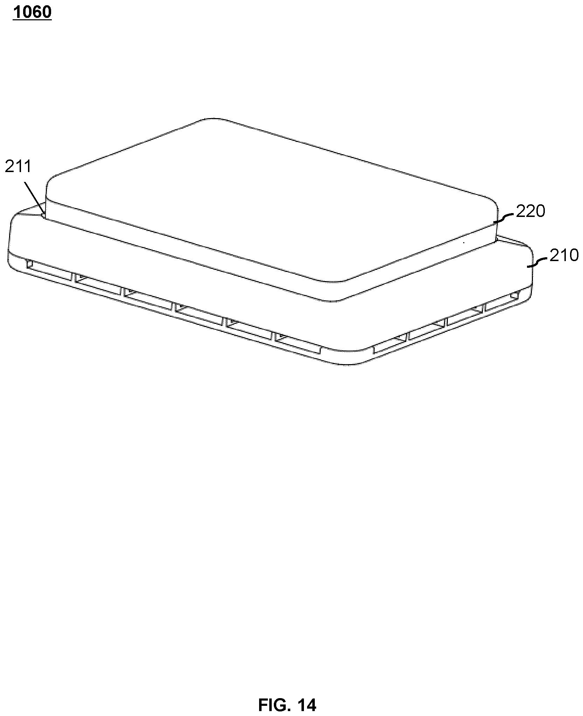

In some embodiments, referring to FIG. 10 and FIG. 14, the wearable device 1000 may also include a fixing seat 1060. The fixing seat 1060 may be coupled to the wearable structure 1020, and the circuit structure 1040 may be disposed in the fixing seat 1060. The fixing seat 1060 is described in detail below in connection with FIG. 14.

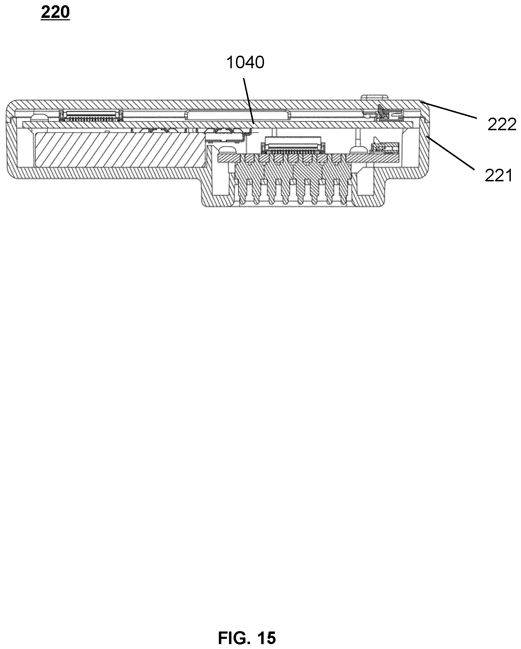

FIG. 14 is a schematic diagram of a structure of a fixing seat according to some embodiments of the present disclosure. FIG. 15 is a schematic diagram of a cross-sectional view of a sub-seat according to some embodiments of the present disclosure.

In some embodiments, as shown in FIG. 14 and FIG. 15, the fixing seat 1060 includes a female seat 210 and a sub-seat 220, the sub-seat 220 is detachably connected to the female seat 210.

The female seat 210 may be disposed on a side of the wearing structure 1020 away from the first electrode 1011 and the second electrode 1012, and the first electrode 1011 and the second electrode 1012 may be electrically connected via the wire 1050 to a circuit structure or a conductive element in the female seat 210. In some embodiments, the female seat 210 may be secured to the wearing structure 1020 by bonding, snap-fitting, embedding, or the like.

In some embodiments, the sub-seat 220 is disposed independently with respect to the female seat 210 and is configured to house the circuit structure 1040. As shown in FIG. 15, the sub-seat 220 includes a first sub-seat housing 221 and a second sub-seat housing 222, the first sub-seat housing 221 and the second sub-seat housing 222 being spliced together to form a body of the sub-seat 220 having a mounting cavity internally with the circuit structure 1040 mounted within the mounting cavity. In some embodiments, the circuit structure 1040 may be mounted in the mounting cavity in the form of a circuit board. In some embodiments, a recess 211 is provided on a side of the female seat 210 away from the wearing structure 1020, and at least a portion of a structure of the sub-seat 220 may be snapped to the female seat 210 through the recess 211, such that the sub-seat 220 is detachably connected to the female seat 210. For example, as shown in FIG. 14, the sub-seat 220 is integrally embedded and snapped in the recess 211 of the female seat 210, realizing the detachable connection between the sub-seat 220 and the female seat 210. As another example, the sub-seat 220 is provided with a protruding structure, which is embedded and snapped in the recess 211 of the female seat 210, realizing the detachable connection between the sub-seat 220 and the female seat 210. In some embodiments, the protruding structure of the sub-seat 220 and the recess 211 of the female seat 210 are provided with a plug and a socket that mate with each other, and the plug and socket are electrically connected when the sub-seat 220 mates with the female seat 210. The ECG signal collected by the first electrode 1011 and the second electrode 1012 may be delivered through the female seat 210 to the circuit structure 1040 of the sub-seat 220 for processing. When the wearable device 1000 needs to be cleaned, the sub-seat 220 may be removed from the female seat 210 to prevent damage to the circuit structure 1040 of the wearable device 1000 during cleaning. In some embodiments, a power supply circuit providing power to the wearable device 1000 may also be provided in the sub-seat 220. It should be noted that, in some embodiments, the sub-seat 220 and the female seat 210 in the fixing seat 1060 may also be a one-piece structure. The fixing seat 1060 may be detachably connected (e.g., bonded, snap-fit, etc.) to the wearing structure 1020, the wire 1050 may be integrated into a connector, and the fixing seat 1060 may be provided with an interface that is mated with the connector. The connector is connected to the interface when the fixing seat 1060 is connected to the sub-seat 220. When the wearable device 1000 needs to be cleaned, the fixing seat 1060 may be disassembled as a whole from the wearing structure 1020 while disconnecting the cooperation between the connector and the interface.

In some embodiments, the wearable device 1000 may also include a sensor module (not shown in the drawing), and the sensor module may be configured to collect movement data of the human body. The sensor module is communicatively connected to the circuit structure 1040. In some embodiments, the sensor module may include a strain sensor, an inertial sensor, a temperature sensor, a humidity sensor, or the like, or any combination thereof. As an exemplary illustration, in some embodiments, the strain sensor may be provided on the wearable structure 1020 to detect a change in a movement direction in a waist region of the human body and send a corresponding detection signal to the circuit structure 1040, and the circuit structure 1040 may predict or direct a movement of the waist region based on the detection signal. In some embodiments, the strain sensor may also detect respiratory information (e.g., respiratory rate, etc.) of the human body based on a change in the undulation of a muscle or skin. In some embodiments, the inertial sensor may be disposed in the circuit structure 1040, e.g., disposed on a circuit board, to detect the movement of the human body and send a corresponding detection signal to the circuit structure 1040. The circuit structure 1040 may determine, based on the detection signal, whether the movement of the human body needs to be adjusted or given guidance, etc. It should be noted that the sensor module is not only applicable to the wearable device 1000, but may also be applicable to the wearable devices provided by the embodiments of the present disclosure, e.g., the wearable device 100 and the wearable device 600.

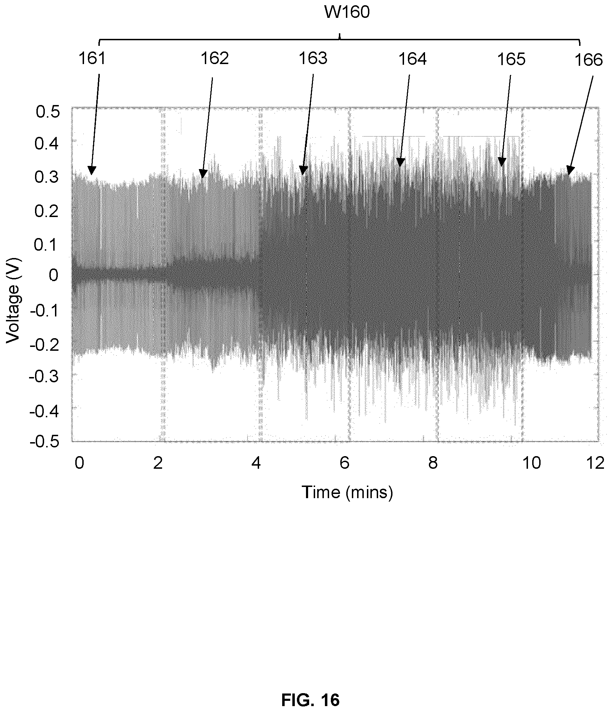

In some embodiments, after conducting a relevant experiment on the wearable devices (e.g., the wearable device 100, the wearable device 600, and the wearable device 1000) provided in the embodiments of the present disclosure, it may be found that the wearable devices provided in the embodiments of the present disclosure can overcome the problem of high interference of motion artifacts on the collected ECG signals when the human body is exercising and ensure that the user can collect high-quality ECG signals when wearing the wearable devices provided in the embodiments of the present disclosure for exercise and fitness (e.g., running), thereby satisfying the demand for heart monitoring. As an exemplary illustration, the experiment may include having an experimental subject wear the wearable device provided in the embodiments of the present disclosure to run on a treadmill at different pacing speeds for a continuous period of time. Then, based on ECG signals of the human body collected by the wearable device at the different pacing speeds, relevant conclusions are drawn, as described in FIGs. 16 to 19 and the related descriptions.

FIG. 16 is a schematic diagram of a waveform of ECG signals of an experimental subject collected over a continuous period of time at different pacing speeds by a wearable device according to some embodiments of the present disclosure. As shown in FIG. 16, the horizontal coordinate represents exercise times (min) of the subject and the vertical coordinate represents ECG signals (V) of the subject at different exercise times. Waveform W160 represents the ECG signals of the subject collected by the wearable device at different pacing speeds over a continuous period of time. Specifically, the waveform W160 may be divided into a first portion 161, a second portion 162, a third portion 163, a fourth portion 164, a fifth portion 165, and a sixth portion 166 based on the pacing speed (or sampling time) of the subject. The first portion 161 corresponds to an ECG signal of the experimental subject collected by the wearable device when the pacing speed is 0 before the experimental subject starts exercising. The second portion 162 corresponds to an ECG signal of the experimental subject collected by the wearable device when the pacing speed is 2. The third portion 163 corresponds to an ECG signal collected by the wearable device when the pacing speed is 6.8. The fourth portion 164 corresponds to an ECG signal collected by the wearable device when the pacing speed is 8.8. The fifth portion 165 corresponds to an ECG signal collected by the wearable device when the pacing speed is 10.8. The sixth portion 166 corresponds to an ECG signal collected by the wearable device when the pacing speed is 0 after the subject finishes exercising. The pacing speed of the treadmill refers to a pacing speed displayed by the treadmill with a unit of kilometers per hour.

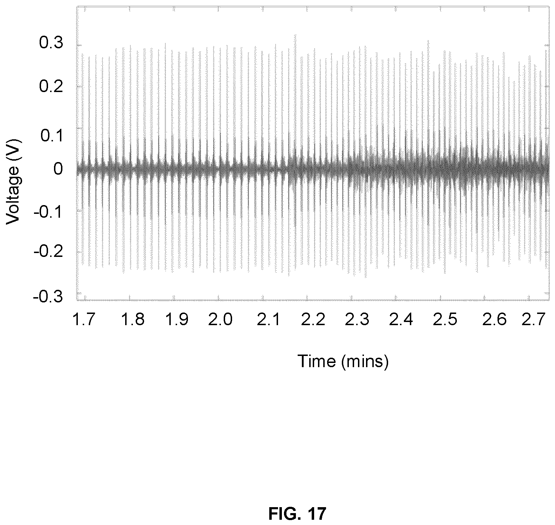

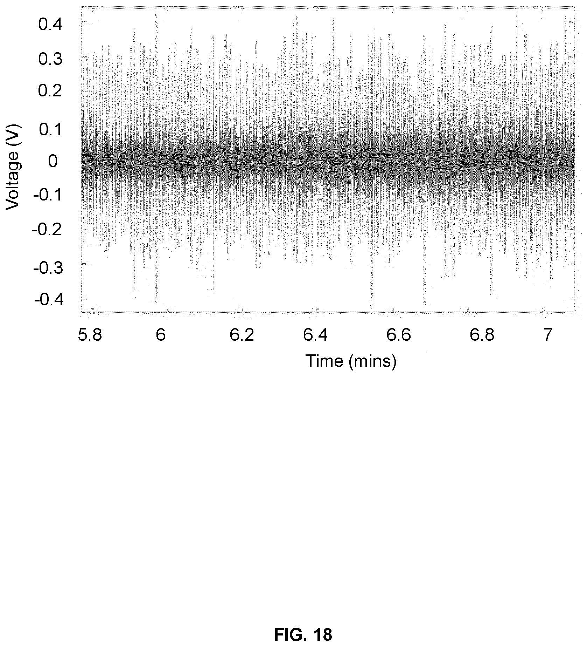

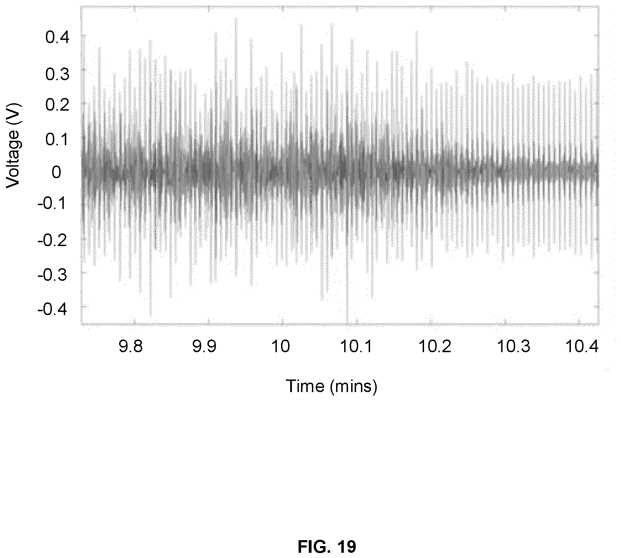

FIG. 17 is an enlarged view of the waveform of the ECG signals shown in FIG. 16 from about 1.7 minutes to 2.7 minutes. FIG. 18 is an enlarged view of the waveform of the ECG signals shown in FIG. 16 from about 5.8 minutes to 7 minutes. FIG. 19 is an enlarged view of the waveform of the ECG signals shown in FIG. 16 from about 9.8 minutes to 10.4 minutes.

Referring to FIGs. 17 to 19, it may be found that even when the experimental subject is running at a relatively high pacing speed, R-wave peaks (e.g., the portion with a relatively light color in the drawings) and motion artifacts (e.g., the portion with a relatively dark color in the drawings) of the waveform of the ECG signals collected by the wearable device provided in the embodiments of the present disclosure may be distinctly differentiated. As the experimental subject runs at a higher pacing speed, although the amplitude of the motion artifacts increases, heart rate information and other features that may reflect the condition of the heart may still be recognized with naked eyes, which shows that the wearable device provided in the present disclosure can overcome the problem of the interference of the motion artifacts to the collection of the ECG signals of the human body during exercise and ensure that the user can collect ECG signals of high quality even when he or she wears the wearable device provided in the embodiments of the present disclosure to perform exercise and fitness (e.g., running), thereby satisfying the demand for heart monitoring.

Embodiments of the present disclosure also provide a cardiac monitoring system that may be combined with the wearable device (e.g., the wearable device 100, the wearable device 600, and the wearable device1000) provided in the embodiments of the present disclosure to perform feature extraction as well as analysis of ECG signals collected by the wearable device for judging, monitoring, early warning, or the like, of the heart condition of the human body. The cardiac monitoring system provided by embodiments of the present disclosure will be described in detail below in conjunction with the accompanying drawings.

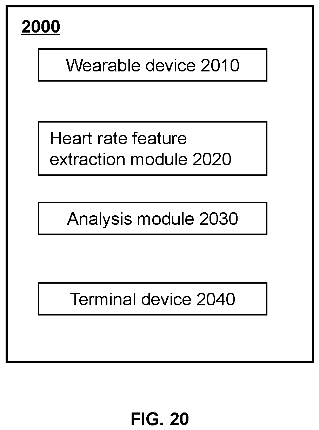

FIG. 20 is a block diagram of a structure of a cardiac monitoring system according to some embodiments of the present disclosure.

As shown in FIG. 20, a cardiac monitoring system 2000 includes a wearable device 2010, a heart rate feature extraction module 2020, and an analysis module 2030.

The wearable device 2010 may be the wearable device provided by embodiments of the present disclosure (e.g., the wearable device 100, the wearable device 600, and the wearable device1000), and the wearable device 2010 may be configured to collect an ECG signal of a human body, and more description of the wearable device 2100 may be found in the relevant descriptions of the wearable device 100, 600, or 1000, which will not be repeated herein.

The heart rate feature extraction module 2020 may be configured to extract a heart rate feature of a human body based on the ECG signal. As an exemplary illustration, the ECG signal collected by the wearable device 2010 may be waveforms such as those illustrated in FIG. 8 or FIG. 9, and the heart rate feature may include features extracted from the ECG signal such as a P wave, a QRS complex, an ST segment, a T wave, a P-R interval, a U wave, a QT interval, or the like. In some embodiments, the heart rate feature extraction module 2200 may process (e.g., denoising of noises such as a motion artifact, a power frequency, a harmonic, or the like) the ECG signal collected by the wearable device 2010 and extract the heart rate feature. In some embodiments, the heart rate feature may include, but is not limited to, any one or more of a heart rate variability feature, a shape, a length, a height, a slope, a trough, a peak of each wave (e.g., the P wave, the R wave, the T wave, the U wave). In some embodiments, the heart rate feature extraction module 2020 may be integrated into a circuit structure of the wearable device 2010 or may be a terminal device (e.g., a smartwatch, a smart helmet, an augmented reality device, a virtual reality device, smart glasses, a cellular phone, a tablet computer, a computer, a fitness device, or the like) that is communicatively connected to the circuit structure of the wearable device 2010.

The analysis module 2030 may be configured to match the heart rate feature with a database of heart rate features to obtain a heart rate analysis result. The heart rate feature may reflect the heart rate, cardiac diseases, cardiac abnormalities, etc., of the human body and the analysis module 2030 may be configured to obtain the heart rate analysis result of the human body by matching the heart rate feature of the human body with the database of heart rate features. As an exemplary illustration only, as shown in connection with FIG. 9, the heart rate of the human body may be calculated by measuring a time interval between two adjacent R waves, and the database of heart rate features may include a preset heart rate range (e.g., 60-100 beats/min). In some embodiments, matching the heart rate feature with the database of heart rate features may include: determining whether the heart rate of the human body is in the preset heart rate range, if the heart rate of the human body is within the preset heart rate range, then the human body has a normal heart rate. If the heart rate is less than a minimum value (e.g., 60 beats/minute) of the preset heart rate range, it indicates bradycardia. If the heart rate is greater than a maximum value (e.g., 100 beats/minute) of the preset heart rate range, it indicates tachycardia. Additionally, a parameter of a heart rate curve between different segments of the ECG signal may reflect a heart health condition of the human body. In some embodiments, the parameter of a heart rate curve may include a shape, a length, a height, a slope, a peak, a trough, or the like, of the curve, or any combination thereof. For example, a level or downward-sloping depression of the ST segment exceeding 0.05 mV may indicate myocardial ischemia in the body, possibly angina. As another example, an elevation of the ST segment exceeding 0.1 mV with dynamic changes, or a deep and wide Q wave, may suggest the presence of acute myocardial infarction. As yet another example, changes in the height of the R and S waves, as well as alterations in the ST segment and T wave, may indicate left ventricular hypertrophy. As a further example, a widened, deformed, and prolonged QRS complex may reflect conduction block in the left or right bundle branch, or conditions such as ventricular enlargement or hypertrophy. In some embodiments, matching the heart rate feature with the database of heart rate features may include matching the parameter of the heart rate curves of the human body in different segments with a predetermined heart rate curve parameter, respectively, to obtain the heart rate analysis result. In some alternative embodiments, matching the heart rate feature with the database of heart rate features may include matching a heart rate signal curve of the human body with a preset heart rate signal curve to obtain the heart rate analysis result, for example, matching the heart rate signal curve directly with heart rate signal curves in a heart rate database to obtain the matching result. In some embodiments, the database of heart rate features may be a pre-established database storing a variety of preset heart rate features and their corresponding heart rate analysis results, wherein the preset heart rate features may be preset heart rate features corresponding to the heart analysis results. As an exemplary illustration, the analysis module 2030 may match the heart rate feature extracted by the heart rate feature extraction module 2020 with the preset heart rate features in the database of heart rate features. If there is a preset heart rate feature in the database of heart rate features that matches the heart rate feature extracted by the heart rate feature extraction module 2020, the analysis module 2030 may obtain the heart rate analysis result corresponding to the preset heart rate feature as the analysis result corresponding to the heart rate feature extracted by the heart rate feature extraction module 2020.

In some embodiments, as shown in FIG. 20, the cardiac monitoring system 2000 may further include a terminal device 2040, and the terminal device 2040 may be configured to receive and display heart rate results. In some embodiments, the terminal device 2040 can include a monitor, which may be configured to receive and display the heart rate analysis result. In some embodiments, the terminal device 2040 may also provide feedback to a user based on the matching result. As an exemplary illustration only, when the user wears the wearable device 2010 for exercise (e.g., running, cycling, etc.), the wearable device 2010 may collect the heart rate feature of the user during the exercise and save the heart rate feature in a storage device of the terminal device 2040 or upload the heart rate feature to a cloud server and form a database of heart rate features for different types of exercise. Heart rate feature data in the database of heart rate features may be heart rate feature data of a particular user during his or her routine exercise, or heart rate feature data of different users during exercise. When a user is exercising normally according to his/her previous habits (e.g., running at a speed within a specific speed range) at a current time, his/her heart rate feature at the current time of exercise may be approximately the same as those of his/her previous exercise, e.g., a curve of heart rate changes at the current time of exercise may approximately coincide with a curve of heart rate changes during the previous exercise. When the user continues to maintain the exercise, a degree of fit of a matching result between the heart rate feature of the user at the current time and a previous heart rate feature (e.g., a heart rate feature in the database of heart rate features) is less than a target value (e.g., 80%, etc.), and the terminal device 2040 may provide feedback to the user based on the matching result. In some embodiments, the terminal device 2040 may include a prompting module, and a processor of the terminal device 2040 may control the prompting module to generate a prompting message to the user based on the matching result. In some embodiments, the prompting message may include any one or more of an audible message (e.g., a voice prompting message, a music prompting message, etc.), a physical stimulation prompting message (e.g., a vibration, a weak electric current stimulation), a textual prompting message, a picture or a video prompting message, an indicator light prompting message, or the like. For example, if the heart rate feature of the user deviates significantly from the preset heart rate features in the database of heart rate features, the prompting module may send a prompting message to the user to pause the current exercise and continuously detect subsequent heart rate changes of the user. If a subsequent heart rate feature of the user deviates significantly from the preset heart rate features in the database of heart rate features, the user may be prompted to pause the exercise plan and be reminded to go to the hospital for corresponding examinations. In some embodiments, the processor of the terminal device 2040 may control the prompting module to make a call or send a message (e.g., rescue information, heart disease information, location information, etc.) to an ambulance center or an emergency contact, based on the matching result. For example, if the heart rate feature of the user is severely abnormal (e.g., arrest, rapid heart rate, or bradycardia), the user is unable to call for help on his or her own at that moment, and the processor of the terminal device 2040 controls the prompting module to automatically make a rescue call or send a rescue message based on that situation. External environmental factors (e.g., temperature, humidity) and the user's own factors (e.g., the intensity of exercise, his or her own disease, etc.) may affect the heart rate feature of the user, and in some embodiments, when analyzing the heart rate feature of the user, a degree of influence of the external environmental factors and the user's own factors on the heart rate feature of the user may be analyzed together. Exemplarily, the greater a difference between an external ambient temperature and a room temperature (e.g., 25°C), the greater the degree of influence on the heart rate feature of the user. The difference may be divided into different intervals, with different intervals corresponding to different influencing factors. An ideal heart rate feature may be obtained based on the actually collected heart rate feature and the influencing factors, and the heart health condition of the user may be analyzed based on the ideal heart rate feature and the preset database of heart rate features. When humidity and the user's own factors affect the heart rate feature of the user, an ideal heart rate feature in this scenario may also be determined in a similar manner, and the heart health condition of the user may be analyzed based on the ideal heart rate feature.

In some embodiments, the terminal device 2040 may be integrated into the wearable device 2010. In some embodiments, the terminal device 2040 may be an external device (e.g., a mobile device, a tablet, a helmet, a smartwatch, smart glasses, a virtual reality device, an augmented reality device, a laptop, and a desktop computer, etc.) that is wired or wirelessly connected to the wearable device 2010.

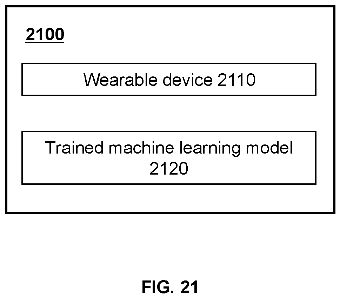

FIG. 21 is a block diagram of a structure of a cardiac monitoring system according to some embodiments of the present disclosure.

As shown in FIG. 21, a cardiac monitoring system 2100 includes a wearable device 2110 and a trained machine learning model 2120. The wearable device 2110 may be configured to collect an ECG signal of a human body, and more descriptions of the wearable device 2110 may be found in the relevant descriptions of the wearable devices 100, 600, or 1000, and will not be repeated herein. The trained machine learning model 2120 may be configured to use the collected ECG signal as an input to output a heart rate analysis result. In some embodiments, the trained machine learning model 2120 may include a K-Nearest Neighbor (KNN) model, a Bayesian model, a decision tree model, a random forest model, a logistic regression model, a neural network (NN) model, an ensemble learning model, or any combination thereof. In some embodiments, the trained machine learning model 2120 may be obtained by training an initial machine learning model. Specifically, as may be known from the relevant descriptions of FIG. 20, there is a correspondence between the ECG signal and the heart rate analysis result, and thus a plurality of ECG signals may be taken as input data for the initial machine learning model and heart rate analysis results corresponding to the plurality of ECG signals may be determined as output data of the initial machine learning model to train the initial machine learning model to obtain the trained machine learning model 2120. It should be noted that the cardiac monitoring system 2100 may also include a terminal device for receiving and displaying the heart rate analysis results.

Having thus described the basic concepts, it may be rather apparent to those skilled in the art after reading this detailed disclosure that the foregoing detailed disclosure is intended to be presented as an illustrative example and is not limiting. Various alterations, improvements, and modifications may occur and are intended for those skilled in the art, though not expressly stated herein. These alterations, improvements, and modifications are intended to be suggested by this disclosure, and are within the spirit and scope of the exemplary embodiments of the present disclosure.

Moreover, certain terminology has been configured to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this disclosure are not necessarily all referring to the same embodiment. Furthermore, the particular features, structures, or characteristics may be combined as suitable in one or more embodiments of the present disclosure.

Similarly, it should be noted that in the foregoing description of embodiments of the present disclosure, various features are sometimes grouped together in a single embodiment, figure, or description thereof for the purpose of streamlining the disclosure aiding in the understanding of one or more of the various inventive embodiments. This way of disclosure, however, is not to be interpreted as reflecting an intention that the claimed subject matter requires more features than are expressly recited in each claim. Rather, inventive embodiments lie in less than all features of a single foregoing disclosed embodiment.

In closing, it is to be understood that the embodiments of the present disclosure disclosed herein are illustrating of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A wearable device, comprising:
at least two electrodes configured to fit against the skin of a human body to collect an electrocardiogram (ECG) signal of the human body; and
a wearable structure configured to carry the at least two electrodes and fit the at least two electrodes to a waist region of the human body, wherein
the at least two electrodes are spaced apart on the wearable structure, the at least two electrodes being disposed on two sides of a mid-sagittal plane of the human body when the human body wears the wearable structure.

2. The wearable device of claim 1, wherein the at least two electrodes include a first electrode and a second electrode, and when the human body wears the wearable structure, the first electrode and the second electrode are fitted to iliac positions on the two sides of the mid-sagittal plane of the human body, respectively.

3. The wearable device of claim 2, wherein when the human body wears the wearable structure, the first electrode and the second electrode are fitted to anterior superior iliac spine positions on the two sides of the mid-sagittal plane of the human body, respectively.

4. The wearable device of claim 2, wherein when the human body wears the wearable structure, the first electrode and the second electrode are fitted to posterior superior iliac spine positions on the two sides of the mid-sagittal plane of the human body, respectively.

5. The wearable device of claim 1, wherein the at least two electrodes include a first electrode and a second electrode, and when the human body wears the wearable structure, the first electrode and the second electrode are fitted to lower back positions on the two sides of the mid-sagittal plane of the human body, respectively.

6. The wearable device of claim 1, wherein the at least two electrodes include a first electrode and a second electrode, and when the human body wears the wearable structure, the first electrode and the second electrode are fitted to abdomen regions on the two sides of the mid-sagittal plane of the human body, respectively.

7. The wearable device of any one of claims 2-6, wherein when the human body wears the wearable structure, the first electrode and the second electrode are disposed symmetrically with respect to the mid-sagittal plane of the human body.

8. The wearable device of claim 7, wherein the wearable structure has a first extension direction and a second extension direction, the second extension direction being perpendicular to the first extension direction, the first electrode and the second electrode are spaced apart along the first extension direction of the wearable structure, a dimension of the first electrode or the second electrode in the first extension direction is in a range of 5 mm ~ 50 mm, and a dimension of the first electrode or the second electrode in the second extension direction is in a range of 5 mm ~ 50 mm.

9. The wearable device of claim 1, wherein the at least two electrodes protrude from a surface of the wearable structure surrounding the at least two electrodes.

10. The wearable device of claim 1, wherein the at least two electrodes include a first electrode, a second electrode, and a reference electrode, the first electrode and the second electrode are spaced apart and disposed on a surface of the wearable structure that is close to the skin of the human body, and the reference electrode is disposed between the first electrode and the second electrode.

11. The wearable device of claim 10, wherein a dimension of the reference electrode along a second extension direction of the wearable structure is not less than a dimension of the first electrode or the second electrode along the second extension direction of the wearable structure.

12. The wearable device of claim 11, wherein the dimension of the reference electrode along the second extension direction is in a range of 5 mm ~ 80 mm.

13. The wearable device of claim 1, further comprising a circuit structure configured to process the ECG signal collected by the at least two electrodes, wherein the circuit structure is disposed on a side of the wearable structure away from the at least two electrodes, and the at least two electrodes are electrically connected to the circuit structure via a wire.

14. The wearable device of claim 13, wherein the circuit structure includes a contact impedance measurement circuit configured to obtain contact impedance when the at least two electrodes are fitted to a surface of the skin.

15. The wearable device of claim 13, further comprising a fixing seat, wherein the fixing seat includes a female seat and a sub-seat, the female seat is connected to the wearable structure, the sub-seat is detachably connected to the female seat, and the circuit structure is located within the sub-seat.

16. The wearable device of claim 13, further comprising a sensor module configured to collect movement data of the human body, the sensor module being communicatively connected to the circuit structure.

17. The wearable device of any one of claims 1-16, wherein the wearable structure is an elastic strap-like structure, a first Velcro patch is provided on a side of the wearable structure in contact with the human body, a second Velcro patch is provided on a side of each of the at least two electrodes away from a side where the electrode is fitted to the human body, and the each of the at least two electrodes is detachably connected with the wearable structure via the first Velcro patch and the second Velcro patch.

18. A cardiac monitoring system, comprising:
the wearable device of any one of claims 1-16, configured to acquire an ECG signal of a human body;
a heart rate feature extraction module configured to extract a heart rate feature of the human body based on the ECG signal;
an analysis module configured to match the heart rate feature with a database of heart rate features to obtain a heart rate analysis result; and
a terminal device configured to receive and display the heart rate analysis result.

19. A cardiac monitoring system, comprising:
the wearable device of any one of claims 1-16, configured to acquire an ECG signal of a human body;
a trained machine learning model configured to output a heart rate analysis result based on the ECG signal as an input; and
a terminal device configured to receive and display the heart rate analysis result.

20. The cardiac monitoring system of claim 18 or 19, wherein the terminal device includes a processor and a prompting module, the processor is configured to issue a command based on the heart rate analysis result, and control the prompting module to send out a prompting message, make a phone call, or send a message.
